# FASCICULE DE BREVET EUROPEEN

(11) **EP 2 331 576 B1**
(45) Date de publication et mention de la délivrance du brevet: **16.07.2014**
(21) Numéro de dépôt: 09737035.7
(22) Date de dépôt: 26.08.2009
(51) Int. Cl.: C07K 16/28, A61K 39/395

(54) **INDUCTION DE L'EXPRESSION DE P53 PAR NEUTRALISATION DE LA NEUROPILINE-2 POUR LE TRAITEMENT DES CANCERS**
INDUKTION DER EXPRESSION VON P53 DURCH NEUTRALISIEREN VON NEUROPILIN-2 ZUR BEHANDLUNG VON KREBS
INDUCTION OF P53 EXPRESSION BY NEUTRALIZATION OF NEUROPILIN-2 FOR THE TREATMENT OF CANCERS

(30) Priorité: 27.08.2008 FR 0804725
(43) Date de publication de la demande: 15.06.2011
(73) Titulaire: Diaclone, 25000 Besançon (FR); Etablissement Français du Sang, 93200 La Plaine Saint-Denis (FR); Université de Franche-Comté, 25000 Besançon (FR)
(72) Inventeur: BORG, Christophe, F-25115 Pouilley les Vignes (FR); WIJDENES, John, F-25720 Larnod (FR); GRAND CLEMENT, Camille, F-25000 Besançon (FR)
(74) Mandataire: Rançon, Xavier Lucien Abel
(86) Numéro de dépôt international: PCT/FR2009/001035
(87) Numéro de publication internationale: WO 2010/023382

(56) Documents cités:
- US-A1- 2006 251 650
- SULPICE ERIC ET AL: "Neuropilin-1 and neuropilin-2 act as coreceptors, potentiating proangiogenic activity." BLOOD 15 FEB 2008, vol. 111, no. 4, 15 février 2008 (2008-02-15), pages 2036-2045, XP002522622 ISSN: 0006-4971
- CAUNT MARESA ET AL: "Blocking neuropilin-2 function inhibits tumor cell metastasis" CANCER CELL, CELL PRESS, US, vol. 13, no. 4, 1 avril 2008 (2008-04-01), pages 331-342, XP002478224 ISSN: 1535-6108
- FAVIER BENOIT ET AL: "Neuropilin-2 interacts with VEGFR-2 and VEGFR-3 and promotes human endothelial cell survival and migration" BLOOD, AMERICAN SOCIETY OF HEMATOLOGY, US, vol. 108, no. 4, 1 août 2006 (2006-08-01), pages 1243-1250, XP002478221 ISSN: 0006-4971
- GRAY MICHAEL J ET AL: "Therapeutic targeting of neuropilin-2 on colorectal carcinoma cells implanted in the murine liver" JOURNAL OF THE NATIONAL CANCER INSTITUTE, OXFORD UNIVERSITY PRESS, GB, vol. 100, no. 2, 1 janvier 2008 (2008-01-01), pages 109-120, XP002478225 ISSN: 0027-8874
- FUTAMURA MANABU ET AL: "Possible role of semaphorin 3F, a candidate tumor suppressor gene at 3p21.3, in p53-regulated tumor angiogenesis suppression." CANCER RESEARCH 15 FEB 2007, vol. 67, no. 4, 15 février 2007 (2007-02-15), pages 1451-1460, XP002522623 ISSN: 0008-5472
- RADER C ET AL: "A phage display approach for rapid antibody humanization: Designed combinatorial V gene libraries", PROCEEDINGS OF THE NATIONAL ACADEMY OF SCIENCES, NATIONAL ACADEMY OF SCIENCES, US, vol. 95, no. 15, 21 July 1998 (1998-07-21) , pages 8910-8915, XP002182776, ISSN: 0027-8424, DOI: 10.1073/PNAS.95.15.8910

### SEQUENCE LISTING

<110> - DIACLONE
   - EFS BOURGOGNE FRANCHE COMTE
   - UNIVERSITE DE FRANCHE COMTE
   BORG, Christophe
   WIJDENES, John
   GRAND CLEMENT, Camille
<120> INDUCTION DE L'EXPRESSION DE P53 PAR NEUTRALISATION DE LA NEUROPILINE-2 POUR LE TRAITEMENT DES CANCERS
<130> MJPDG884-10W
<150> FR0804725
   <151> 2008-08-27
<160> 32
<170> PatentIn version 3.3
<210> 1
   <211> 349
   <212> DNA
   <213> Mus musculus
<220>
   <221> CDS
   <222> (1)..(348)
<220>
   <221> V_region
   <222> (1)..(294)
<220>
   <221> misc_feature
   <222> (1)..(75)
   <223> FR1
<220>
   <221> misc_feature
   <222> (76)..(99)
   <223> CDR1
<220>
   <221> misc_feature
   <222> (100)..(150)
   <223> FR2
<220>
   <221> misc_feature
   <222> (151)..(174)
   <223> CDR2
<220>
   <221> misc_feature
   <222> (175)..(288)
   <223> FR3
<220>
   <221> misc_feature
   <222> (286)..(318)
   <223> JUNCTION
<220>
   <221> misc_feature
   <222> (289)..(315)
   <223> CDR3
<220>
   <221> misc_feature
   <222> (316)..(349)
   <223> FR4
<400> 1
   gaa gtt aag ctg cag gag tca ggg gca gag ctt gtg aag cca ggg gcc      48
   Glu Val Lys Leu Gln Glu Ser Gly Ala Glu Leu Val Lys Pro Gly Ala
   1               5                   10                  15
   tca gtc aag ttg tcc tgc aca gtt tct ggc ttc aac att aaa gac acc      96
   Ser Val Lys Leu Ser Cys Thr Val Ser Gly Phe Asn Ile Lys Asp Thr
               20                  25                  30
   tat ata cac tgg gtg ata cag agg cct gaa cag ggc ctg gag tgg ctt     144
   Tyr Ile His Trp Val Ile Gln Arg Pro Glu Gln Gly Leu Glu Trp Leu
           35                  40                  45
   gga agg att gat cct gcg aat ggt aat act aaa tat gac ccg aag ttc     192
   Gly Arg Ile Asp Pro Ala Asn Gly Asn Thr Lys Tyr Asp Pro Lys Phe
       50                  55                  60
   cag ggc aag gcc act ata aca gca gac aca tcc tcc aac aca gcc tac     240
   Gln Gly Lys Ala Thr Ile Thr Ala Asp Thr Ser Ser Asn Thr Ala Tyr
   65                  70                  75                  80
   ctg cag ctc agc agc ctg acc tct gag gac act gcc gtc tat tac tgt     288
   Leu Gln Leu Ser Ser Leu Thr Ser Glu Asp Thr Ala Val Tyr Tyr Cys
                   85                  90                  95
   gct aga tgg gcg gtt gta ggt gac tac tgg ggc caa ggc acc act ctc     336
   Ala Arg Trp Ala Val Val Gly Asp Tyr Trp Gly Gln Gly Thr Thr Leu
               100                 105                 110
   aca gtc tcc tca g                                                   349
   Thr Val Ser Ser
           115
<210> 2
   <211> 116
   <212> PRT
   <213> Mus musculus
<400> 2
   Glu Val Lys Leu Gln Glu Ser Gly Ala Glu Leu Val Lys Pro Gly Ala
   1 5 10 15
   Ser Val Lys Leu Ser Cys Thr Val Ser Gly Phe Asn Ile Lys Asp Thr
   20 25 30
   Tyr Ile His Trp Val Ile Gln Arg Pro Glu Gln Gly Leu Glu Trp Leu
   35 40 45
   Gly Arg Ile Asp Pro Ala Asn Gly Asn Thr Lys Tyr Asp Pro Lys Phe
   50 55 60
   Gln Gly Lys Ala Thr Ile Thr Ala Asp Thr Ser Ser Asn Thr Ala Tyr
   65 70 75 80
   Leu Gln Leu Ser Ser Leu Thr Ser Glu Asp Thr Ala Val Tyr Tyr Cys
   85 90 95
   Ala Arg Trp Ala Val Val Gly Asp Tyr Trp Gly Gln Gly Thr Thr Leu
   100 105 110
   Thr Val Ser Ser
   115
<210> 3
   <211> 318
   <212> DNA
   <213> Mus musculus
<220>
   <221> V_region
   <222> (1)..(283)
<220>
   <221> misc_feature
   <222> (1)..(77)
   <223> FR1
<220>
   <221> CDS
   <222> (3)..(317)
<220>
   <221> misc_feature
   <222> (78)..(95)
   <223> CDR1
<220>
   <221> misc_feature
   <222> (96)..(146)
   <223> FR2
<220>
   <221> misc_feature
   <222> (147)..(155)
   <223> CDR2
<220>
   <221> misc_feature
   <222> (156)..(263)
   <223> FR3
<220>
   <221> misc_feature
   <222> (261)..(290)
   <223> JUNCTION
<220>
   <221> misc_feature
   <222> (264)..(287)
   <223> CDR3
<220>
   <221> misc_feature
   <222> (288)..(318)
   <223> FR4
<400> 3
   ga tat tgt gat cac cca ctc tac aaa ttc ctg cat gta tca gca gga        47
      Tyr Cys Asp His Pro Leu Tyr Lys Phe Leu His Val Ser Ala Gly
      1               5                   10                  15
   gac agg gtt acc ata acc tgc aag gcc agt cag agt gtg agt gat gat       95
   Asp Arg Val Thr Ile Thr Cys Lys Ala Ser Gln Ser Val Ser Asp Asp
                   20                  25                  30
   gtg gct tgg tac caa cag aag cca ggg cag tct cct aaa ctg ctg ata      143
   Val Ala Trp Tyr Gln Gln Lys Pro Gly Gln Ser Pro Lys Leu Leu Ile
               35                  40                  45
   tac tct gca tcc aat cgc tac act gga gtc cct gat cgc ttc act ggc      191
   Tyr Ser Ala Ser Asn Arg Tyr Thr Gly Val Pro Asp Arg Phe Thr Gly
           50                  55                  60
   agt gga tat ggg acg gat ttc act ttc acc atc agc act gtg cag cct      239
   Ser Gly Tyr Gly Thr Asp Phe Thr Phe Thr Ile Ser Thr Val Gln Pro
       65                  70                  75
   gaa gac ctg gca gtt tat ttc tgt cag cag gat tat agc tct ccc acg      287
   Glu Asp Leu Ala Val Tyr Phe Cys Gln Gln Asp Tyr Ser Ser Pro Thr
   80                  85                  90                  95
   ttc ggt tct ggg acc aag ctg gag ctg aaa c                            318
   Phe Gly Ser Gly Thr Lys Leu Glu Leu Lys
                   100                 105
<210> 4
   <211> 105
   <212> PRT
   <213> Mus musculus
<400> 4
   Tyr Cys Asp His Pro Leu Tyr Lys Phe Leu His Val Ser Ala Gly Asp
   1               5                   10                  15
   Arg Val Thr Ile Thr Cys Lys Ala Ser Gln Ser Val Ser Asp Asp Val
               20                  25                  30
   Ala Trp Tyr Gln Gln Lys Pro Gly Gln Ser Pro Lys Leu Leu Ile Tyr
           35                  40                  45
   Ser Ala Ser Asn Arg Tyr Thr Gly Val Pro Asp Arg Phe Thr Gly Ser
       50                  55                  60
   Gly Tyr Gly Thr Asp Phe Thr Phe Thr Ile Ser Thr Val Gln Pro Glu
   65                  70                  75                  80
   Asp Leu Ala Val Tyr Phe Cys Gln Gln Asp Tyr Ser Ser Pro Thr Phe
                   85                  90                  95
   Gly Ser Gly Thr Lys Leu Glu Leu Lys
               100                 105
<210> 5
   <211> 358
   <212> DNA
   <213> Mus musculus
<220>
   <221> CDS
   <222> (1)..(357)
<220>
   <221> V_region
   <222> (1)..(295)
<220>
   <221> misc_feature
   <222> (1)..(75)
   <223> FR1
<220>
   <221> misc_feature
   <222> (76)..(99)
   <223> CDR1
<220>
   <221> misc_feature
   <222> (100)..(150)
   <223> FR2
<220>
   <221> misc_feature
   <222> (151)..(174)
   <223> CDR2
<220>
   <221> misc_feature
   <222> (175)..(288)
   <223> FR3
<220>
   <221> misc_feature
   <222> (286)..(327)
   <223> JUNCTION
<220>
   <221> misc_feature
   <222> (289)..(324)
   <223> CDR3
<220>
   <221> misc_feature
   <222> (325)..(358)
   <223> FR4
<400> 5
   gag gtg cag ctg gag gag tca ggg gga ggc tta gtg aag cct gga ggg        48
   Glu Val Gln Leu Glu Glu Ser Gly Gly Gly Leu Val Lys Pro Gly Gly
   1               5                   10                  15
   tcc ctg aaa ctc tcc tgt gca gcc tct gga ttc act ttc agt gac tat        96
   Ser Leu Lys Leu Ser Cys Ala Ala Ser Gly Phe Thr Phe Ser Asp Tyr
               20                  25                  30
   tac atg tat tgg gtt cgc cag act ccg gaa aag agg ctg gag tgg gtc       144
   Tyr Met Tyr Trp Val Arg Gln Thr Pro Glu Lys Arg Leu Glu Trp Val
           35                  40                  45
   gca acc att agt gat ggt ggt agt tac acc tac tat cca gac agt att       192
   Ala Thr Ile Ser Asp Gly Gly Ser Tyr Thr Tyr Tyr Pro Asp Ser Ile
       50                  55                  60
   aag ggc cga ttc acc atc tcc agg gac aat gcc agg aac aac ctg tac       240
   Lys Gly Arg Phe Thr Ile Ser Arg Asp Asn Ala Arg Asn Asn Leu Tyr
   65                  70                  75                  80
   ctt caa atg agc agt ctg aag tct gag gac aca gcc atg tat tac tgt       288
   Leu Gln Met Ser Ser Leu Lys Ser Glu Asp Thr Ala Met Tyr Tyr Cys
                   85                  90                  95
   gca aga ggt ggg ccc tat agg tcc tgg ttt gct ttc tgg ggc caa ggg       336
   Ala Arg Gly Gly Pro Tyr Arg Ser Trp Phe Ala Phe Trp Gly Gln Gly
               100                 105                 110
   act ctg gtc act gtc tct gca g                                         358
   Thr Leu Val Thr Val Ser Ala
           115
<210> 6
   <211> 119
   <212> PRT
   <213> Mus musculus
<400> 6
   Glu Val Gln Leu Glu Glu Ser Gly Gly Gly Leu Val Lys Pro Gly Gly
   1 5 10 15
   Ser Leu Lys Leu Ser Cys Ala Ala Ser Gly Phe Thr Phe Ser Asp Tyr
   20 25 30
   Tyr Met Tyr Trp Val Arg Gln Thr Pro Glu Lys Arg Leu Glu Trp Val
   35 40 45
   Ala Thr Ile Ser Asp Gly Gly Ser Tyr Thr Tyr Tyr Pro Asp Ser Ile
   50 55 60
   Lys Gly Arg Phe Thr Ile Ser Arg Asp Asn Ala Arg Asn Asn Leu Tyr
   65 70 75 80
   Leu Gln Met Ser Ser Leu Lys Ser Glu Asp Thr Ala Met Tyr Tyr Cys
   85 90 95
   Ala Arg Gly Gly Pro Tyr Arg Ser Trp Phe Ala Phe Trp Gly Gln Gly
   100 105 110
   Thr Leu Val Thr Val Ser Ala
   115
<210> 7
   <211> 337
   <212> DNA
   <213> Mus musculus
<220>
   <221> CDS
   <222> (1)..(336)
<220>
   <221> V_region
   <222> (1)..(302)
<220>
   <221> misc_feature
   <222> (1)..(78)
   <223> FR1
<220>
   <221> misc_feature
   <222> (79)..(111)
   <223> CDR1
<220>
   <221> misc_feature
   <222> (112)..(162)
   <223> FR2
<220>
   <221> misc_feature
   <222> (163)..(171)
   <223> CDR2
<220>
   <221> misc_feature
   <222> (172)..(279)
   <223> FR3
<220>
   <221> misc_feature
   <222> (277)..(309)
   <223> JUNCTION
<220>
   <221> misc_feature
   <222> (280)..(306)
   <223> CDR3
<220>
   <221> misc_feature
   <222> (307)..(337)
   <223> FR4
<400> 7
   gat att gtg atc acc cag act cca ctc tcc ctg cct gtc agt ctt gga        48
   Asp Ile Val Ile Thr Gln Thr Pro Leu Ser Leu Pro Val Ser Leu Gly
   1               5                   10                  15
   gat caa gcc tcc atc tct tgc aga tct agt cag agc att gtg tat agt        96
   Asp Gln Ala Ser Ile Ser Cys Arg Ser Ser Gln Ser Ile Val Tyr Ser
               20                  25                  30
   aat gga aac acc tat tta gaa tgg tac ctg cag aaa cca ggc cag tct       144
   Asn Gly Asn Thr Tyr Leu Glu Trp Tyr Leu Gln Lys Pro Gly Gln Ser
           35                  40                  45
   cca aag ctc ctg atc tac aaa gtt tcc aac cga ttt tct ggg gtc cca       192
   Pro Lys Leu Leu Ile Tyr Lys Val Ser Asn Arg Phe Ser Gly Val Pro
       50                  55                  60
   gac agg ttc agt ggc agt gga tca ggg aca gat ttc aca ctc aag atc       240
   Asp Arg Phe Ser Gly Ser Gly Ser Gly Thr Asp Phe Thr Leu Lys Ile
   65                  70                  75                  80
   agc aga gtg gag gct gag gat ctg gga gtt tat tac tgc ttt caa ggt       288
   Ser Arg Val Glu Ala Glu Asp Leu Gly Val Tyr Tyr Cys Phe Gln Gly
                   85                  90                  95
   tca cat gtt cct ccg acg ttc ggt gga ggc acc aag ctg gaa atc aaa c     337
   Ser His Val Pro Pro Thr Phe Gly Gly Gly Thr Lys Leu Glu Ile Lys
               100                 105                 110
<210> 8
   <211> 112
   <212> PRT
   <213> Mus musculus
<400> 8
   Asp Ile Val Ile Thr Gln Thr Pro Leu Ser Leu Pro Val Ser Leu Gly
   1 5 10 15
   Asp Gln Ala Ser Ile Ser Cys Arg Ser Ser Gln Ser Ile Val Tyr Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Pro Thr Phe Gly Gly Gly Thr Lys Leu Glu Ile Lys
   100 105 110
<210> 9
   <211> 8
   <212> PRT
   <213> Mus musculus
<400> 9
   Gly Phe Asn Ile Lys Asp Thr Tyr
   1 5
<210> 10
   <211> 8
   <212> PRT
   <213> Mus musculus
<400> 10
   Ile Asp Pro Ala Asn Gly Asn Thr
   1 5
<210> 11
   <211> 9
   <212> PRT
   <213> Mus musculus
<400> 11
   Ala Arg Trp Ala Val Val Gly Asp Tyr
   1 5
<210> 12
   <211> 6
   <212> PRT
   <213> Mus musculus
<400> 12
   Gln Ser Val Ser Asp Asp
   1 5
<210> 13
   <211> 3
   <212> PRT
   <213> Mus musculus
<400> 13
   Ser Ala Ser
   1
<210> 14
   <211> 8
   <212> PRT
   <213> Mus musculus
<400> 14
   Gln Gln Asp Tyr Ser Ser Pro Thr
   1 5
<210> 15
   <211> 8
   <212> PRT
   <213> Mus musculus
<400> 15
   Gly Phe Thr Phe Ser Asp Tyr Tyr
   1 5
<210> 16
   <211> 8
   <212> PRT
   <213> Mus musculus
<400> 16
   Ile Ser Asp Gly Gly Ser Tyr Thr
   1 5
<210> 17
   <211> 12
   <212> PRT
   <213> Mus musculus
<400> 17
   Ala Arg Gly Gly Pro Tyr Arg Ser Trp Phe Ala Phe
   1 5 10
<210> 18
   <211> 11
   <212> PRT
   <213> Mus musculus
<400> 18
   Gln Ser Ile Val Tyr Ser Asn Gly Asn Thr Tyr
   1 5 10
<210> 19
   <211> 3
   <212> PRT
   <213> Mus musculus
<400> 19
   Lys Val Ser
   1
<210> 20
   <211> 9
   <212> PRT
   <213> Mus musculus
<400> 20
   Phe Gln Gly Ser His Val Pro Pro Thr
   1 5
<210> 21
   <211> 23
   <212> DNA
   <213> Artificial Sequence
<220>
   <223> PCR PRIMER
<400> 21
   gargttaagc tgsaggagtc agg 23
<210> 22
   <211> 27
   <212> DNA
   <213> Artificial Sequence
<220>
   <223> PCR PRIMER
<400> 22
   atagacagat gggggtgtcg ttttggc 27
<210> 23
   <211> 23
   <212> DNA
   <213> Artificial Sequence
<220>
   <223> PCR PRIMER
<400> 23
   gaggtgcagc tggaggagtc agg 23
<210> 24
   <211> 27
   <212> DNA
   <213> Artificial Sequence
<220>
   <223> PCR PRIMER
<400> 24
   atagacagat gggggtgtcg ttttggc 27
<210> 25
   <211> 24
   <212> DNA
   <213> Artificial Sequence
<220>
   <223> PCR PRIMER
<400> 25
   gatattgtga tsacmcardc taca 24
<210> 26
   <211> 22
   <212> DNA
   <213> Artificial Sequence
<220>
   <223> PCR PRIMER
<400> 26
   ggatacagtt ggtgcagcat ta 22
<210> 27
   <211> 24
   <212> DNA
   <213> Artificial Sequence
<220>
   <223> PCR PRIMER
<400> 27
   gatattgtgm tsacccagac tcca 24
<210> 28
   <211> 22
   <212> DNA
   <213> Artificial Sequence
<220>
   <223> PCR PRIMER
<400> 28
   ggatacagtt ggtgcagcat ta 22
<210> 29
   <211> 23
   <212> DNA
   <213> Homo sapiens
<400> 29
   aaaggctgga agtcagcact aat 23
<210> 30
   <211> 23
   <212> DNA
   <213> Homo sapiens
<400> 30
   aaaaattagt gctgacttcc agc 23
<210> 31
   <211> 22
   <212> RNA
   <213> Artificial
<220>
   <223> siRNA
<400> 31
   ggcuggaagu cagcacuaau uu 22
<210> 32
   <211> 22
   <212> RNA
   <213> Artificial
<220>
   <223> siRNA
<400> 32
   auuagugcug acuuccagcc uu 22

## Description

La présente demande est relative au traitement du cancer par surexpression du gène suppresseur des tumeurs p53 et induction de l'apoptose des cellules tumorales par ciblage de la neuropiline 2.

Le cancer est caractérisé par une prolifération cellulaire incontrôlée généralement due à des mutations au niveau des gènes contrôlant positivement (les oncogènes) ou négativement (les gènes suppresseurs des tumeurs) la prolifération cellulaire. De par leur prolifération anarchique, les cellules tumorales envahissent localement le tissu sain, et après avoir subi de nouvelles mutations, peuvent acquérir la faculté de migrer dans la circulation sanguine et de se propager à distance de la tumeur initiale pour former des métastases. Cependant, la néovascularisation de la tumeur via le mécanisme d'angiogénèse est essentielle à la croissance tumorale, puisque toute cellule, et en particulier les cellules tumorales très exigeantes en oxygène et en énergie, ne peuvent survivre à plus de quelques dizaines de millimètres d'un vaisseau sanguin.

Les traitements anticancéreux actuels ont donc pour cibles principales, d'une part les cellules tumorales elles-mêmes, et d'autre part l'angiogénèse tumorale.

En ce qui concerne les stratégies anti-angiogénèse, l'implication des facteurs de croissance, notamment l'EGF ou le VEGF (Vascular Endothelial Growth Factor), dans la progression des cancers et dans l'angiogenèse a été mise en évidence. Plusieurs molécules ciblant l'action des VEGFs ont été développées comme médicament anti-angiogénèse : le bevacizumab (Avastin®), anticorps monoclonal humanisé dirigé contre le VEGF, a été utilisé dès 2004 pour le traitement du cancer colorectal métastatique ; le sunitinib et le sorafenib, molécules inhibant la transduction du signal de la voie VEGF/VEGF-Receptor, ont été utilisés dans des stratégies anti-angiogénèse pour le traitement du carcinome rénal métastatique.

Concernant les stratégies ciblant la tumeur et les cellules tumorales, l'ablation des tissus tumoraux par voie chirurgicale (lorsque cela est possible), la radiothérapie des tumeurs solides, des immunothérapies visant à renforcer la réponse immunitaire contre les cellules cancéreuses, et les chimiothérapies représentent les traitements thérapeutiques standards. Les avancées majeures dans la compréhension des mécanismes moléculaires de la cancérogénèse ont permis le développement de nouvelles chimiothérapies, dont l'efficacité dépend cependant du type de cancer à traiter et de l'organe atteint. En outre, de nombreux cancers posent encore des problèmes de santé publique majeurs et s'avèrent résistants aux thérapeutiques classiques. Par exemple, les cancers digestifs (pancréas, cholangiocarcinomes, cancers colorectaux) deviennent au cours de leur histoire naturelle, rapidement résistants à la chimiothérapie. La perte du gène suppresseur des tumeurs codant pour la protéine p53 est un des mécanismes majeurs expliquant la résistance des cellules tumorales à l'apoptose et aux traitements anti-néoplasiques conventionnels comme la chimiothérapie ou la radiothérapie. En effet, la protéine p53 a pour fonction d'arrêter le cycle cellulaire ou d'induire l'apoptose d'une cellule en réponse aux dommages de l'ADN cellulaire ou à l'activation des oncogènes. La protéine p53 joue donc un rôle central dans le contrôle du cycle cellulaire et dans le maintien de l'intégrité du génome, en permettant à la cellule dont le cycle cellulaire est interrompu de réparer l'anomalie génétique, ou bien en entrainant sa destruction par apoptose. p53 étant essentielle pour protéger l'organisme des effets d'une division cellulaire aberrante ou incontrôlée, l'absence de p53, sa sous-expression ou l'expression d'une protéine p53 non-fonctionnelle, a pour conséquence la survie des cellules tumorales.

Dans un modèle d'étude *in vitro* et dans des modèles murins, il a été montré que la restauration de l'expression de p53 par transfert de gène favorise la régression des cancers et améliore l'efficacité des traitements cytotoxiques. Des études *in vitro* ont également montré que dans des cellules exprimant une protéine sauvage et une protéine p53 mutante ayant un effet dominant négatif envers la protéine sauvage, l'interférence par un siARN ciblant spécifiquement l'ARNm codant pour la protéine p53 mutante rétablit la fonction de p53 (MARTINEZ et al. P.N.A.S., 99(23): 14849-54, 2002). Il a par ailleurs été montré qu'il est possible de "réactiver" des protéines p53 mutantes en stabilisant une conformation fonctionnelle de ces protéines. Les molécules CP-31398, PRIMA-1 et MIRA-1, premières molécules développées présentant cette capacité, ont été utilisées avec succès dans un modèle de xénogreffe humaine pour inhiber la croissance tumorale (pour revue, cf. par exemple LEVESQUE & EASTMAN, Carcenogenesis., 28(1):13-20, 2007).

La restauration de l'expression d'une protéine p53 fonctionnelle, ainsi que l'induction de la surexpression de cette protéine, représentent donc des objectifs essentiels dans le développement de thérapies antitumorales.

Les Inventeurs ont maintenant mis en évidence l'existence d'une corrélation inverse entre la neuropiline-2 (NRP-2) et l'expression de la protéine p53, et ont montré que de façon surprenante l'expression de p53 pouvait être induite ou augmentée en inhibant l'expression de la neuropiline-2 par siARN.

La neuropiline-2, glycoprotéine transmembranaire d'environ 130 kDa, est un récepteur des sémaphorines, notamment de la sémaphorine 3F, et de facteurs de croissance de la famille du VEGF. Elle est exprimée chez l'homme par les neurones, les cellules endothéliales, les ostéoblastes et par une large variété de néoplasmes. Elle est composée d'un domaine intracytoplasmique de 40 acides aminés environ, d'un domaine transmembranaire et d'un domaine extracellulaire. Ce domaine extracellulaire comprend un domaine A, constitué de 2 sous-domaines (ala2), un domaine B également constitué de 2 sous-domaines (b1b2), et un domaine C. Il a été montré que le domaine B constituait le site de liaison du VEGF, alors que la liaison avec la sémaphorine 3F impliquait à la fois le domaine A et le domaine B (GERETTI et al., J. Biol. Chem., 282, 25698-707, 2007) ; le domaine C est quant à lui impliqué dans l'oligomérisation de la NRP-2.

Dans de nombreux cas, les neuropilines sont les seuls récepteurs des VEGF exprimés par les cellules cancéreuses (BIELENBERG et al. Exp. Cell. Res., 312(5) :584-593, 2006), et plusieurs études ont montré que l'expression, voire la surexpression des neuropilines, est généralement corrélée à une augmentation de la croissance tumorale et du caractère invasif et métastatique des cancers, ainsi qu'à un pronostic défavorable.

En outre, de nombreuses observations indiquent que ces récepteurs jouent un rôle crucial dans la progression tumorale en activant l'angiogénèse : notamment, la liaison du VEGF à la NRP-2 est responsable d'une activité pro-angiogénique, médiée par une coopération entre le court domaine intracytoplasmique de NRP-2 et celui du récepteur au VEGF VEGFR1. Il a été rapporté que NRP-2 a la capacité d'induire la phosphorylation du récepteur VEGFR1 et de la protéine AKT, favorisant ainsi la progression des cancers, et que la suppression de la neuropiline-2 par siARN s'oppose à l'apparition de métastases dans des xénogreffes et réduit la taille des tumeurs (GRAY et al. J. Natl. Cancer Inst., 100 :109-120, 2008). Il a par ailleurs été montré qu'un anticorps anti-NPR-2 (dénommé anti-Nrp2^{B}), dirigé contre le site de liaison au VEGF du domaine B de NRP-2, pouvait réduire la lymphangiogenèse tumorale et la formation de métastases ; cet anticorps agit en inhibant la migration des cellules endothéliales lymphatiques, mais n'a aucun effet sur la migration, la prolifération, ou l'apoptose des cellules tumorales (CAUNT et al., Cancer Cell, 13, 331-42, 2008).

Les inventeurs ont généré des anticorps anti-NRP-2, et ont constaté que certains d'entre eux produisaient les mêmes effets sur l'expression de p53 et l'induction de l'apoptose des cellules tumorales que l'inhibition de NRP-2 par siARN, et que ces effets étaient indépendants du VEGF. En outre, ils ont constaté *in vitro* et *in vivo* dans un modèle murin que ces anticorps potentialisaient l'efficacité de traitements anticancéreux.

Ces données ouvrent de nouvelles perspectives thérapeutiques, en particulier concernant la possibilité de développer des stratégies innovantes de sensibilisation à la chimiothérapie ou à la radiothérapie ou à d'autres agents anti-cancéreux.

Ainsi, la présente invention a pour objet un anticorps anti-neuropiline-2 humaine, ou un ligand de la neuropiline-2 humaine dérivé dudit anticorps, caractérisé en ce que sa fixation à des cellules tumorales exprimant la neuropiline 2 humaine induit l'apoptose desdites cellules tumorales.

Avantageusement, des anticorps ou ligands anti-neuropiline 2 humaine conformes à l'invention possèdent, outre leur capacité d'induction de l'apoptose des cellules tumorales exprimant la neuropiline 2 humaine, les caractéristiques suivantes :
- ils induisent l'expression de p53, et leur capacité à induire l'apoptose est dépendante de cette expression de p53 (elle est diminuée par un inhibiteur de p53, la pifithrin-α) ;
- leurs propriétés de liaison à la neuropiline, et leur capacité à induire l'apoptose sont indépendantes du VEGF (leur fixation à la surface de cellules tumorales exprimant la neuropiline-2 humaine et leur capacité à induire l'apoptose desdites cellules ne sont pas modifiées par la présence de VEGF).

Des anticorps peuvent ainsi être sélectionnés, à partir d'anticorps anti-neuropiline 2 humaine ou de ligands dérivés de ceux-ci, sur la base de leur capacité à induire l'apoptose de cellules tumorales exprimant la neuropiline-2 humaine, et/ou sur la base d'une ou plusieurs des autres caractéristiques mentionnées ci-dessus.

Les anticorps peuvent être des anticorps naturels, polyclonaux ou monoclonaux, des anticorps recombinants, notamment des anticorps chimériques ou humanisés. On entend par « anticorps chimérique » un anticorps possédant les domaines variables de l'anticorps monoclonal dont il est issu, couplés aux domaines constants d'un autre anticorps, de préférence un anticorps humain.

Le terme « anticorps humanisé » se réfère à un anticorps initialement produit par un animal non humain, de préférence la souris, ayant conservé sa spécificité de liaison à la neuropiline 2, mais dans lequel, afin de réduire son immunogénicité chez l'homme, le plus de séquences murines possible ont été remplacées par les séquences humaines correspondantes. En ce qui concerne les domaines variables, les séquences remplacées sont en général les régions FR (framework), c'est-à-dire les séquences situées entre les boucles hypervariables CDRs.

Des anticorps chimériques ou humanisés sont de préférence des immunoglobulines de classe IgG, et notamment d'isotypes IgG1, 2, 3 ou 4.

On entend par « ligand de la neuropiline 2 humaine dérivé d'un anticorps anti-neuropiline 2 », tout ligand de la neuropiline 2 comprenant au moins les CDR3 de la chaine lourde et ceux de la chaîne légère dudit anticorps, et de préférence comprenant également les CDR2 et/ou les CDR1 de la chaine lourde et de la chaîne légère dudit anticorps.

Des ligands de la neuropiline 2 conformes à l'invention peuvent être en particulier :
- tout fragment d'un anticorps anti-neuropiline-2 conforme à l'invention comprenant les CDR3, et en outre les CDR2 et les CDR1 des chaînes lourde et légère dudit anticorps.

Des fragments d'anticorps anti-neuropiline 2 sont notamment des fragments Fv, dsFv, Fab, Fab'2 ou scFv. Les fragments Fv sont constitués des domaines variables des chaînes lourde et légère VH et VL d'un anticorps, associés l'un à l'autre par des interactions hydrophobes. Le fragment dsFv est constitué d'un dimère VH :: VL relié par un pont disulfure. Les fragments scFv sont constitués des portions variables des chaînes lourdes et légères d'un anticorps, reliées entre elles par l'intermédiaire d'un lieur flexible (CLACKSON et al., Nature, 352: 624-628, 1991), formant ainsi une protéine simple-chaîne. Les fragments Fab résultent de l'action de la papaïne sur une molécule d'immunoglobuline, et contiennent chacun une chaîne légère et la première moitié d'une chaîne lourde reliées entre elles par un pont disulfure. Le fragment F(ab')2 peut être obtenu par traitement d'un anticorps par la pepsine : ce fragment comprend deux fragments Fab et une partie de la région charnière. Les fragments Fab' peuvent être obtenus à partir des fragments F(ab')2 par coupure du pont disulfure dans la région charnière.

Ces fragments se liant à l'antigène peuvent également être combinés afin d'obtenir des dérivés plurivalents, tels que les « diabodies » ou « triabodies », résultant de l'association de 2 ou 3 de ces fragments se liant à l'antigène.

Des protéines recombinantes comprenant un fragment d'anticorps anti-neuropiline-2 conforme à l'invention peuvent être notamment :
- des protéines associant au moins un fragment d'anticorps anti-neuropiline-2 conforme à l'invention avec au moins un fragment d'un autre anticorps ; on citera à titre d'exemples, des immunoglobulines bi-spécifiques, des conjugués d'un fragment Fv ou Fab d'un anticorps anti-neuropiline-2 avec un fragment Fv ou Fab d'un anticorps de spécificité différente, des « diabodies bi-spécifiques » résultant de l'association d'un fragment scFv d'un anticorps anti-neuropiline-2 avec un fragment Fv ou Fab d'un anticorps de spécificité différente ;
- des protéines associant au moins un fragment d'anticorps anti-neuropiline-2 conforme à l'invention avec une molécule permettant de prolonger sa demi-vie plasmatique lors de son administration *in vivo,* notamment avec un polypeptide hydrosoluble de masse moléculaire suffisante pour que la masse moléculaire du polypeptide de fusion ainsi obtenu soit supérieure au seuil de filtration rénale.

Des anticorps chimériques ou recombinants, des fragments scFv et leurs dérivés, etc. peuvent être obtenus par les techniques classiques du génie génétique, telles que celles décrites par SAMBROOK et al. (MOLECULAR CLONING, A LABORATORY MANUAL, 2nd Ed., Cold Spring Harbor Laboratory Press, Cold Spring Harbor, N.Y., 1989).

Des polynucléotides codant les régions variables d'un anticorps anti-neuropiline 2 conforme à l'invention peuvent être obtenus par clonage desdites régions à partir d'une banque d'ADNc d'un hybridome produisant ledit anticorps. Ils peuvent également être préparés totalement ou partiellement par synthèse d'acides nucléiques, à partir des séquences nucléotidiques desdites régions variables.

Diverses méthodes pour obtenir des anticorps humanisés sont également bien connues en elles-mêmes (pour revue cf. par exemple ALMAGRO & FRANSSON, Frontiers in Bioscience, 13, 1619-1633, 2008).

On peut citer les méthodes basées sur la greffe de CDRs qui consiste à transférer les CDRs d'un anticorps non-humain dans les régions de charpente (FR pour « framework régions ») d'un anticorps d'origine humaine (cf. par exemple ROUTLEDGE et al., "Reshaping antibodies for therapy", in Protein Engineering of Antibody Molecules for Prophylatic and Therapeutic Applications in Man, 13-44, Academic Titles, Nottingham, England, 1993, ou ROGUSKA et al., Protein Engineering, 9(10): 895-904, 1996). La greffe de CDRs est généralement complétée par l'optimisation des régions de charpente, qui consiste à modifier quelques résidus des régions de charpente afin d'augmenter l'affinité de liaison à l'antigène de l'anticorps humanisé. L'utilisation de librairies combinatoires permet de simplifier cette étape d'optimisation (ROSOK. et al. J. Biol. Chem. 271: 22611-22618, 1996; BACA et al. J. Biol. Chem. 272: 10678-10684, 1997). Une autre stratégie d'humanisation d'anticorps consiste à conserver uniquement les CDR3 des chaines lourdes et légères de l'anticorps d'origine, et à sélectionner le reste de la séquence à partir de banques naïves de gènes V humains (RADER et al., Proc. Natl. Acad. Sci. U.S.A. 95: 8910-8915, 1998).

Deux exemples d'anticorps monoclonaux anti-neuropiline-2 humaine conformes à l'invention, sont les anticorps ITAC-B1 et ITAC-B2 décrits ci-après. Ces anticorps monoclonaux ont été sélectionnés par les Inventeurs, sur la base de leur capacité à induire l'apoptose de cellules tumorales exprimant NRP-2.

Les séquences de la chaîne lourde et de la chaîne légère d'ITAC-B1 et d'ITAC-B2, ont été déterminées. Ces séquences, ainsi que les séquences polypeptidiques déduites, sont représentées dans le Tableau 1 ci-après (pour la chaîne légère et la chaîne lourde d'ITAC-B1) et dans le Tableau 2 ci-après (pour la chaine légère et la chaîne lourde d'ITAC-B2). Les séquences nucléotidiques sont également représentées dans la liste de séquences en annexe respectivement sous les numéros SEQ ID NO:1, 3, 5 et 7, et les séquences polypeptidiques sont également représentées respectivement sous les numéros SEQ ID NO:2, 4, 6 et 8.

L'hybridome CNCM I-4054, produisant l'anticorps ITAC-B1 a d'autre part été déposé, selon le Traité de Budapest, le 30 juillet 2008 auprès de la Collection Nationale de Culture de Microorganismes (Institut Pasteur, 25 rue du Docteur Roux, 75724 Paris Cedex 15, France, sous le numéro CNCM I-4054.

**TABLEAU 1**

| **ITAC-B1** | |
|---|---|
| **Chaîne lourde (Région V**-**D**-**J**) | |
| **Séquence nucléotidique** | |
| **Séquence peptidique** | |

| Chaîne légère (Région V-J) | |
|---|---|
| **Séquence nucléotidique** | |
| **Séquence peptidique** | |

**TABLEAU 2**

| **ITAC-B2** | |
|---|---|
| **Chaîne lourde (Région V-D-J)** | |
| **Séquence nucléotidique** | |
| **Séquence peptidique** | |

| Chaîne légère (Région V-J) | |
|---|---|
| **Séquence nucléotidique** | |
| **Séquence peptidique** | |

Les séquences codant les CDRs d'ITAC-B1 et ITAC-B2 ont également été déterminées, à partir des séquences des chaînes lourdes et des chaînes légères ci-dessus, à l'aide du logiciel IMGT/V-QUEST (GIUDICELLI et al., Nucleic Acids Research 32, W435-W440, 2004). Les séquences polypeptidiques déduites sont représentées ci après dans le Tableau 3 pour l'anticorps ITAC-B1, et le tableau 4 pour l'anticorps ITAC-B2. Elles sont également représentées dans la liste de séquences en annexe sous les numéros SEQ ID NO: 9 à 20.

**TABLEAU 3**

| ITAC-B1 | Séquence |
|---|---|
| Chaîne lourde | |
| VH-CDR1 | GFNIKDTY (SEQ ID NO:9) |
| VH-CDR2 | IDPANGNT (SEQ ID NO:10) |
| VH-CDR3 | ARWAWGDY (SEQ ID NO:11) |
| Chaîne légère | |
| VL-CDR1 | QSVSDD (SEQ ID NO:12) |
| VL-CDR2 | SAS (SEQ ID NO:13) |
| VL-CDR3 | QQDYSSPT (SEQ ID NO:14) |

**TABLEAU 4**

| ITAC-B2 | Séquence |
|---|---|
| Chaîne lourde | |
| VH-CDR1 | GFTFSDYY (SEQ ID NO:15) |
| VH-CDR2 | ISDGGSYT (SEQ ID NO:16) |
| VH-CDR3 | ARGGPYRSWFAF (SEQ ID NO:17) |
| Chaîne légère | |
| VL-CDR1 | QSIVYSNGNTY (SEQ ID NO:18) |
| VL-CDR2 | KVS (SEQ ID NO:19) |
| VL-CDR3 | FQGSHVPPT (SEQ ID NO:20) |

Les anticorps comprenant les domaines variables d'ITAC-B1 (c'est-à-dire comprenant une chaîne lourde dont le domaine variable est défini par la séquence SEQ ID NO: 2 et une chaîne légère dont le domaine variable est défini par la séquence SEQ ID NO: 4) ou les domaines variables d'ITAC-B2 (c'est-à-dire comprenant une chaîne lourde dont le domaine variable est défini par la séquence SEQ ID NO: 6 et une chaîne légère dont le domaine variable est défini par la séquence SEQ ID NO: 8), constituent des modes de réalisation préférés de l'objet de la présente invention.

La présente demande a également pour objet tout polynucléotide codant pour un anticorps conforme à l'invention ou pour un ligand de la neuropiline 2 humaine dérivé dudit anticorps, ainsi qu'un vecteur recombinant, notamment un vecteur d'expression contenant ledit nucléotide. La présente demande a également pour objet des cellules produisant des anticorps ou des dérivés d'anticorps conformes à l'invention. Il peut s'agir notamment d'hybridomes, par exemple l'hybridome CNCM I-4054, et de cellules-hôtes transformées par un vecteur d'expression. Ladite cellule-hôte peut être une cellule procaryote ou eucaryote. Parmi les cellules eucaryotes utilisables, on citera en particulier des cellules végétales, des cellules de levure, telles que *Saccharomyces,* des cellules d'insecte, telles que les cellules de *Drosophila,* ou de *Spodoptera* et des cellules de mammifères telles que les cellules HeLa, CHO, 3T3, C127, BHK, COS, etc...

La construction de vecteurs d'expression, et la transformation des cellules-hôtes peut être effectuée par les techniques classiques de biologie moléculaire.

La présente invention a également pour objet un anticorps anti-neuropiline-2 humaine conforme à l'invention, ou un ligand de la neuropiline-2 humaine dérivé dudit anticorps pour l'utilisation comme médicament, notamment comme médicament anti-tumoral.

Selon un mode de réalisation préféré de la présente invention, ledit médicament est destiné à induire l'apoptose de cellules tumorales exprimant la neuropiline 2, notamment en augmentant l'expression de p53 dans lesdites cellules tumorales.

L'invention est applicable à tous les types de tumeurs exprimant la neuropiline-2, et notamment aux cancers colorectaux, aux cancers du sein, au cancer du rein, et aux mélanomes. L'expression de la neuropiline-2 dans une tumeur peut aisément être détectée, par exemple à l'aide d'anticorps anti-neuropiline-2 humaine.

Pour la mise en oeuvre de la présente invention, l'anticorps ou le ligand de la neuropiline-2 humaine conforme à l'invention peut être administré par voie intraveineuse, intra-artérielle ou intra-péritonéale.

De manière avantageuse, il peut être administré en combinaison avec un autre agent anti-tumoral.

Des agents antitumoraux utilisables en combinaison avec un anticorps ou un ligand de la neuropiline-2 humaine conforme à l'invention sont en particulier les agents de chimiothérapie (de type alkylants, analogues nucléotidiques ou inhibiteurs de topoisomérases), des radiations ionisantes ou des biothérapies (notamment des molécules de thérapeutiques ciblées neutralisant par exemple le VEGF,ou le récepteur à l'EGF, des inhibiteurs de tyrosine kinases ou des inhibiteurs de la voie mTor).

La présente invention sera mieux comprise à l'aide du complément de description qui va suivre, qui se réfère à des exemples non-limitatifs illustrant l'effet de l'inhibition de NRP-2 par siRNA sur l'expression de p53, et l'apoptose des cellules tumorales, et la préparation d'anticorps anti-NRP-2 reproduisant ces effets.

### EXEMPLE 1 : GENERATION DE LIGNEES CELLULAIRES PRESENTANT LA NEUROPILINE-2 HUMAINE A LEUR SURFACE MEMBRANAIRE

Deux lignées cellulaires ont été transfectées afin d'exprimer NRP-2 à leur surface. Une lignée murine, le mastocytome P815 (Diaclone), tumeur expérimentale largement utilisée comme modèle en immunologie tumorale, et qui n'exprime pas naturellement la NRP-2 humaine, ainsi qu'une lignée tumorale humaine, élaborée à partir de cellules cancéreuses colorectales HT29 n'exprimant pas naturellement la NRP-2 à sa surface membranaire, ont été transfectées par un vecteur d'expression de la NRP-2 humaine. Le vecteur d'expression utilisé, basé sur un plasmide pcDNA3.1 (Invitrogen) a été décrit par Rossignol M et al, (Genomics. 2000 Dec 1;70(2):211-22).

La lignée murine p815 transfectée a été utilisée pour la production d'anticorps monoclonaux murins dirigés contre la glycoprotéine NRP-2 humaine, la lignée humaine transfectée a ensuite été utilisée lors d'expériences fonctionnelles évaluant l'intérêt des anticorps anti-NRP-2.

La transfection des lignées a été réalisée à l'aide d'un kit Effecten® (Qiagen). Les cellules mastocytaires P815 et tumorales HT29 ont été cultivées dans 20mL de milieu DMEM jusqu'à une concentration de 200000 cellules/mL. Les cellules ont alors été lavées une fois avec 20mL de PBS et mises à une concentration de 2x10⁵ cellules/mL dans 4mL de milieu DMEM en flasque. Les cellules ont alors été transfectées avec 1µg de vecteur (vecteur plasmidique à 1µg/µL dans du tampon TBE).

Les cellules P815 et HT29 transfectées ont été laissées en petites flasques et mises à l'incubateur à 37°C sous 5% de CO₂ dans une atmosphère humidifiée, pendant 48h. A J3, les milieux de cultures ont été changés par un nouveau milieu comprenant 0,8 mg/mL de geneticine (G418, Invitrogen, France), pour la sélection des cellules transfectées.

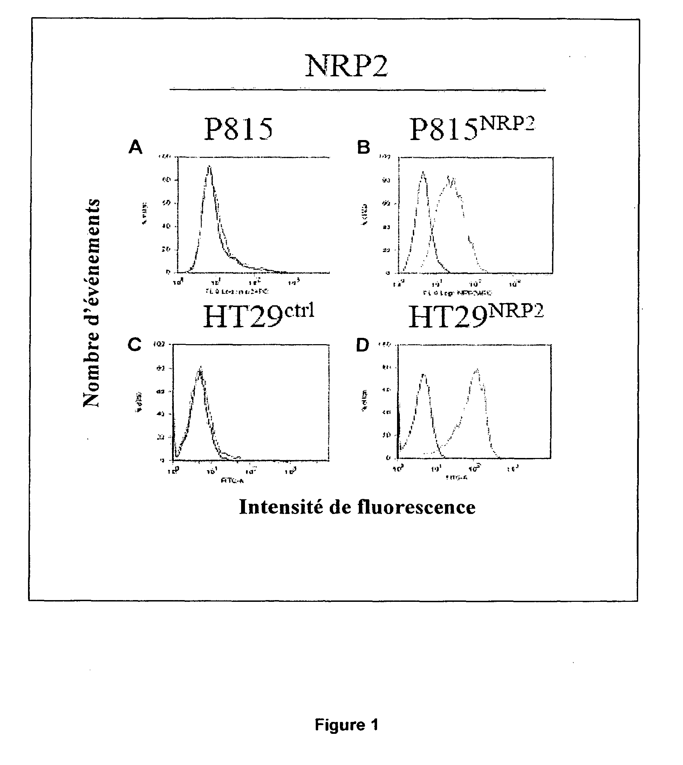

Les cellules efficacement transfectées et exprimant NRP-2 à leur surface sont nommées P815-NRP-2 et HT29-NRP-2. L'efficacité de la transfection est évaluée à J7 par marquage membranaire avec un anticorps IgG murin anti-NRP-2 humaine (Clone C9, Santa Cruz Biotechnology) puis lecture en cytométrie en flux. Le résultat de l'étude en cytométrie de flux est représenté à la Figure 1.

Les panneaux A et C (P815 et HT29ctr) de la Figure 1 représentent les résultats obtenus par cytométrie en flux avec les cellules contrôles alors que les panneaux B et D (P815-NRP-2 et HT29-NRP-2) représentent les résultats obtenus avec des cellules exprimant NRP-2 à leur surface membranaire. Les courbes de distribution en traits noirs représentent les résultats du marquage avec un anticorps contrôle, les courbes de distribution en traits gris représentent les résultats du marquage avec l'anticorps IgG murin anti-NRP-2 humaine.

Pour chacun des panneaux de la Figure 1, le nombre d'évènements (nombre de cellules) est indiqué en ordonnée, l'intensité de fluorescence (correspondant au marquage des cellules par l'anticorps IgG murin anti-NRP-2 humaine) est représentée en abscisse. Les panneaux A et C (P815 et HT29ctrl) de la Figure 1 montrent que les pics de distribution représentant la population cellulaire P815 et HT29ctrl (témoins non transfectées) en contact avec l'anticorps anti-NRP-2 humaine (trait gris) ou avec un anticorps IgG contrôle (trait noir) se superposent : ceci signifie que les cellules P815 et HT29ctrl (témoins non transfectées) ne sont pas davantage marquées par l'anticorps anti-NRP-2 humaine que par un anticorps IgG contrôle et qu'elles n'expriment donc pas NRP-2. En revanche, lorsque les cellules P815 et HT29 transfectées par un vecteur d'expression de la NRP-2 humaine (panneaux B et D, P815-NRP-2 et HT29-NRP-2 respectivement) sont mises en présence de l'anticorps anti-NRP-2 humaine, il y a un net déplacement du pic de distribution de la population cellulaire vers la droite (pic trait gris) par rapport au marquage avec l'anticorps IgG contrôle (pic trait noir). Ces résultats attestent que les cellules transfectées expriment bien NRP-2 à leur surface membranaire.

### EXEMPLE 2: PRODUCTION ET CARACTÉRISATION D'ANTICORPS MONOCLONAUX ANTI-NRP-2 HUMAINE

Un protocole d'immunisation dérivé de celui décrit par MATTHEW et SANDROCK (J. Immunol. Methods, 100: 73-82, 1987) a été utilisé. Dans chaque experience, cinq souris Balb/C femelles (Charles River Laboratories) ont été immunisées avec des cellules transfectées P815-NRP-2 une fois par semaine pendant 5 semaines.

Chaque immunisation consiste en l'administration en « foot-pad » de 1x10⁶ cellules P815-NRP-2 dans chaque patte postérieure de la souris (soit 2x10⁶ P815-NRP-2 par souris). Les cellules servant à l'immunisation d'une souris ont été diluées dans 25µL de PBS 1X et dans 25µL d'adjuvant Ribi (Immunochem Research, USA). 50µL de mélange cellulaire ont alors été injectés à chaque souris (25µL par patte postérieure) à plusieurs reprises durant le protocole d'immunisation. Cinq jours après la dernière injection, les ganglions des souris ont été prélevés et les lymphocytes ont été fusionnés avec une lignée de myélome. La fusion a été effectuée de la façon suivante : les lymphocytes prélevés ont été fusionnés avec des cellules de myélome murin X63/AG 8653 (le rapport lymphocytes/cellules de myélome est de 5 :1), en présence de polyéthylène glycol (KEARNEY et al, J. of Immunol., 123: 1548, 1978). Le myélome murin P3X63/AG8.653 provient de l'ATCC (ref CRL-1580).

La suspension de cellules fusionnées a été lavée une fois, et cultivée sur un milieu sélectif composé de 500 mL de milieu RPMI 1640 (Sigma, France), additionné de 10% de SVF inactivé par la chaleur (Abcys, France), de 4 mM de L-glutamine (Sigma, France), 100 µg/mL de streptomycine, de 100 UI/ml de pénicilline (Sigma, France), de 13,6 µg/mL d'hypoxanthine, 0,19 µg/mL d'aminoptérine et 3.88 UI /mL de thymidine (solution 50X, Sigma, France). Ce milieu ne permet ni la survie des cellules myélomateuses n'ayant pas fusionné, puisque celles-ci sont incapables de synthétiser l'inositol monophosphate, ni la survie des lymphocytes qui n'ont pas la capacité de se multiplier indéfiniment *in vitro.* En revanche les hybridomes survivent car ils présentent d'une part la capacité de métaboliser l'hypoxanthine exogène (propriété des lymphocytes), et d'autre part ont la capacité de se multiplier indéfiniment (« immortalité ») des cellules X63/AG 8653.

Dix jours après la fusion, les surnageants des cultures dans lesquelles une croissance d'hybridomes a été observée ont été testés pour détecter la production d'anticorps monoclonaux anti-NRP-2. Dans ce but, les surnageants de chaque puits de culture d'hybridomes ont été testés par cytométrie en flux sur les lignées cellulaires exprimant ou non la neuropiline 2.

Les hybridomes produisant des anticorps reconnaissant la lignée P815-NRP-2 ont été clonés en utilisant la méthode de la dilution limite (densité d'ensemencement de 1 cellule par puits de culture).

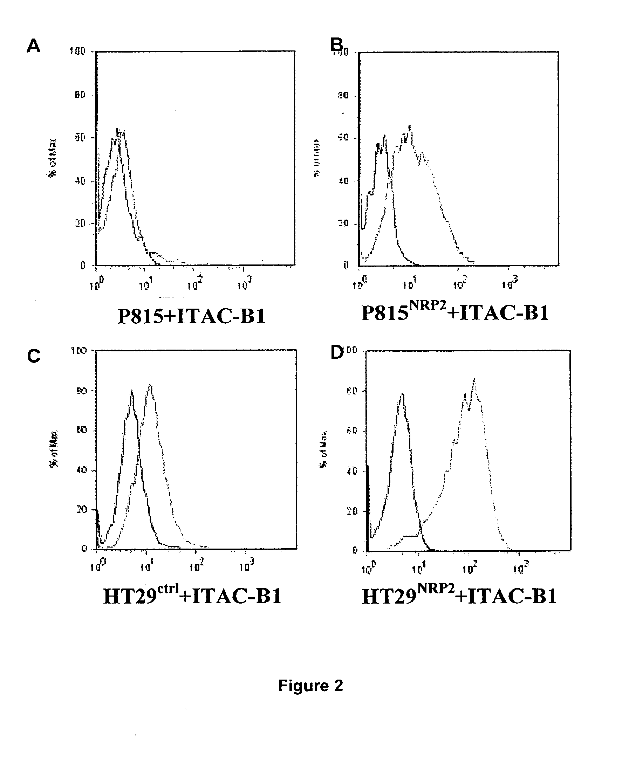

Plusieurs candidats reconnaissant spécifiquement NRP-2 ont été générés et sélectionnés pour leur capacité à induire une apoptose des cellules tumorales exprimant la neuropiline 2, dont les clones ITAC-B1 et ITAC-B2. Le clone ITAC-B1 a été déposé selon le Traité de Budapest, auprès de la CNCM (Collection Nationale des Microorganisme, Institut Pasteur, 25 rue du Docteur Roux, 75724 Paris Cedex 15, France) le 30 juillet 2008 sous le numéro de dépôt CNCM I-4054. Afin de caractériser les anticorps monoclonaux ITAC-B1 et ITAC-B2, un marquage membranaire a été réalisé par cytométrie en flux en mettant en présence 200000 cellules HT29, HT29-NRP-2, P815 ou P815-NRP-2 avec 5µg/mL d'ITAC-B1 ou d'ITAC-B2, 15 minutes, à 4°C. Un anticorps secondaire de chèvre anti-souris couplé à du FITC a été incubé ensuite 15 minutes dans le noir, à 4°C, avant lecture par cytométrie en flux. Les résultats de l'analyse par cytométrie de flux obtenus pour ITAC-B1 sont représentés à la figure 2.

Pour chacun des panneaux de la Figure 2, le nombre d'évènements (nombre de cellules) est indiqué en ordonnée, l'intensité de fluorescence (correspondant au marquage des cellules par l'anticorps ITAC-B1) est représentée en abscisse. Les panneaux A et C (P815 + ITAC-B1 et HT29ctr + ITAC-B1) de la Figure 2 représentent les résultats obtenus par cytométrie en flux avec les cellules contrôle alors que les panneaux B et D (P815-NRP-2 + ITAC-B1 et HT29-NRP-2 + ITAC-B1) représentent les résultats obtenus avec des cellules exprimant NRP-2 à leur surface membranaire. Les courbes de distribution en traits noirs représentent les résultats du marquage avec un anticorps contrôle, les courbes de distribution en traits gris représentent les résultats du marquage avec les anticorps ITAC-B1. On constate, pour les lignées cellulaires exprimant NRP-2, un net déplacement vers la droite du pic de distribution des populations cellulaire lors du marquage par ITAC-B1 (trait gris) en comparaison du marquage avec l'anticorps IgG contrôle (trait noir). Pour les lignées cellulaires n'exprimant pas NRP-2 ce déplacement du pic de distribution est très faible. Ces résultats montrent que les anticorps ITAC-B1 reconnaissent spécifiquement la neuropiline-2 présente à la surface membranaire.

Des résultats similaires ont été obtenus pour l'anticorps ITAC-B2.

Les séquences génomiques de la région variable des chaînes lourdes des hybridomes produisant les anticorps ITAC-B1 et ITAC-B2 ont été analysées par séquençage respectivement avec les amorces :
- 5'-GARGTTAAGCTGSAGGAGTCAGG- 3' (amorce sens dégénérée) (SEQ ID NO : 21)
- 5'-ATAGACAGATGGGGGTGTCGTTTTGGC- 3' (amorce anti-sens) (SEQ ID NO : 22) ; et
- 5'-GAGGTGCAGCTGGAGGAGTCAGG- 3' (amorce sens) (SEQ ID NO : 23)
- 5'-ATAGACAGATGGGGGTGTCGTTTTGGC- 3' (amorce anti-sens) (SEQ ID NO : 24).

Les séquences génomiques de la région variable des chaînes lourdes des hybridomes produisant les anticorps ITAC-B1 et ITAC-B2 ont été analysées par séquençage respectivement avec les amorces :
- 5'-GATATTGTGATSACMCARDCTACA- 3' (amorce sens dégénérée) (SEQ ID NO : 25)
- 5'-GGATACAGTTGGTGCAGCATTA- 3' (amorce anti-sens) (SEQ ID NO : 26);
   et
- 5'-GATATTGTGMTSACCCAGACTCCA- 3' (amorce sens dégénéré) (SEQ ID NO : 27)
- 5'-GGATACAGTTGGTGCAGCATTA- 3' (amorce anti-sens) (SEQ ID NO : 28).

Les séquences d'acides aminés des boucles hypervariables CDR1, CDR2 et CDR3 des régions variables des anticorps ITAC-B1 et ITAC-B2 ont été déterminées à l'aide du logiciel IMGT/V-QUEST version 3.0.0 sur la base de données d'immunoglobulines de « the international ImMunoGeneTics information system® » (IMGT/GENE-DB).

### EXEMPLE 3: ROLE DE LA NEUROPILINE-2 DANS LA PROLIFERATION CELLULAIRE

### Production d'un vecteur exprimant des siARN double brin ciblant le gène de la NRP-2 humaine

Un oligonucléotide sens 5'-AAA GGC TGG AAG TCA GCA CTA AT-3' (SEQ ID NO : 29), et un oligonucléotide anti-sens : 5'-AAA AAT TAG TGC TGA CTT CCA GC-3' (SEQ ID NO : 30) correspondant à une partie de la séquence génique de la NRP-2 humaine ont été hybridés, et le duplex résultant a été inséré dans un vecteur d'expression double promoteur (pFiv H1/U6puro SiARN Expression vector, System Biosciences) préalablement digéré par l'enzyme BbsI.

Après avoir vérifié que l'insert avait bien la taille attendue (21 paires de bases), 100µL de bactéries compétentes E. Coli HB101 (Gibco) ont été transformées par 1µg du vecteur contenant cet insert. Une colonie a été amplifiée dans 200mL de milieu LB avec ampicilline en utilisant un Hi-speed midi prep kit (Qiagen), puis une maxiprep (Qiagen) a été réalisée et a conduit à la purification du plasmide pFiv H1/U6puro SiARN-NRP-2.

Puisque le plasmide pFiv H1/U6puro SiARN-NRP-2 contient les promoteurs H1 et U6 de l'ARN polymérase III bordant l'insert, chacun des brins de l'insert est transcrit dans les cellules transfectées par ce plasmide, aboutissant à la génération de siARN double brin dirigé contre les transcrits NRP-2, constitué de:
Brin sens : 5'- GGC UGG AAG UCA GCA CUA AUU U -3' (SEQ ID NO : 31);
Brin anti-sens : 5'- AUU AGU GCU GAC UUC CAG CCU U - 3' (SEQ ID NO : 32);

### Production d'une lignée cellulaire exprimant un siARN ciblant le gène de la NRP-2 humaine

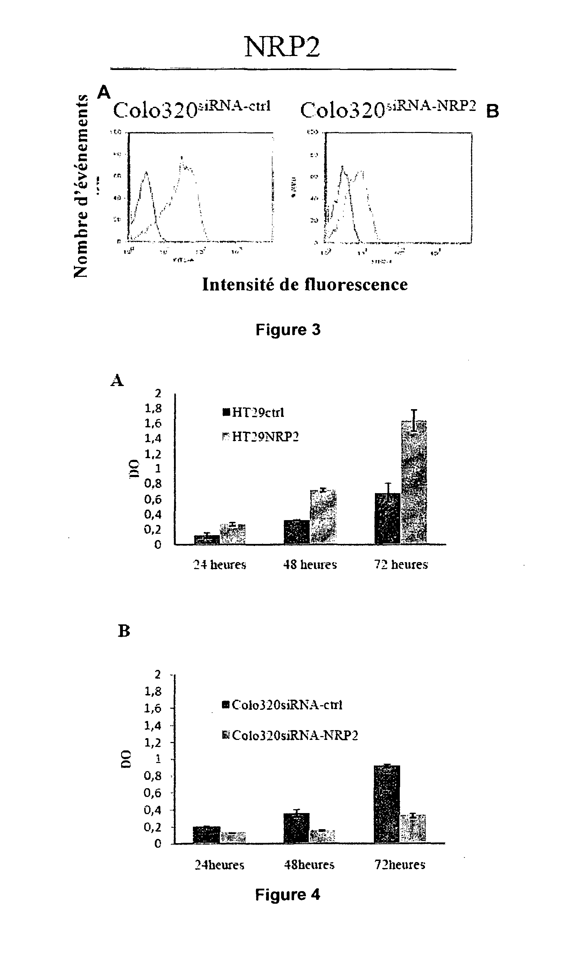

La lignée Colo320 exprimant naturellement la Neuropiline 2 à sa surface membranaire a été transfectée par le plasmide pFiv H1/U6puro siARN-NRP-2 en utilisant le kit Effectene®, Qiagen). Parallèlement, des cellules Colo320 ont été transfectées par un siARN contrôle (siARN-ctrl) fourni avec le kit pFiv H1/U6puro SiARN Expression vector (System Biosciences). Les cellules transfectées Colo320^{siARN-NRP-2} et Colo320^{siARN-ctrl} ont ensuite été sélectionnées à J2 par 2µg/mL de puromycine. L'efficacité de la transfection a été évaluée dès J7 par marquage avec un anticorps IgG murin anti-NRP-2 humaine (Clone C9, Santa Cruz Biotechnology) en cytométrie en flux : les résultats obtenus sont représentés à la Figure 3.

Pour chacun des panneaux de la Figure 3, le nombre d'évènement (le nombre de cellules) est indiqué en ordonnée, l'intensité de fluorescence (correspondant au marquage des cellules par l'anticorps anti-NRP-2 C9) est représentée en abscisse. Les courbes de distribution en traits noirs représentent les résultats obtenus après marquage avec un anticorps contrôle, les courbes de distribution en traits gris représentent les résultats obtenus après marquage avec l'anticorps C9. Le panneau B de la Figure 3, qui représente l'analyse réalisée avec les cellules Colo320^{siARN-NRP-2} exprimant naturellement NRP-2, montre un déplacement vers la gauche du pic de distribution des cellules marquées par l'anticorps C9, par rapport à l'analyse réalisée avec les cellules Colo320^{siARN-ctrl} qui est représentée sur le panneau A.

Ces résultats montrent que l'expression de la protéine NRP-2 est efficacement inhibée dans les cellules exprimant le siARN-NRP-2.

### Test de prolifération en présence du siARN-NRP-2 : test MTT

Le test de prolifération MTT se base sur la réduction du bromure de 3-(4,5-dimethylthiazol-2-yl)-2,5-diphenyl tetrazolium), par la succinate déshydrogénase mitochondriale des cellules vivantes actives, en formazan. L'intensité de la coloration (DO) induite par cette réaction est proportionnelle au nombre de cellules vivantes présentes lors du test, et à leur activité métabolique.

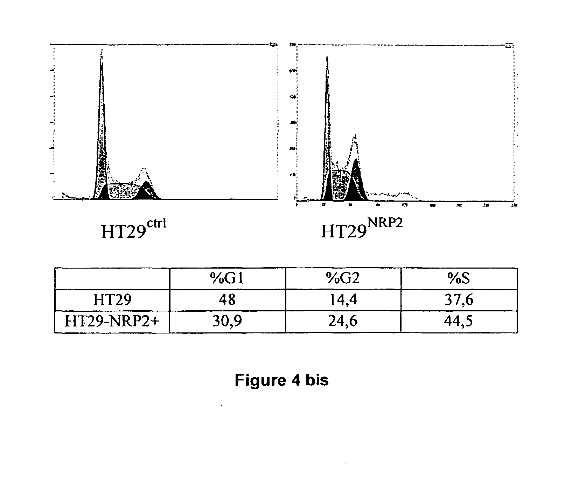

4000 cellules HT29ctrl, HT29-NRP-2, Colo320^{siARN-ctrl} ou Colo320^{siARN-NRP-2} ont été ensemencées dans une plaque Maxisorp 96 puits, dans 100µL de milieu DMEM contenant 10% de SVF inactivé. Le test a été effectué en triplicate. A 24, 48 et 72 heures respectivement, 10 µL de MTT à 5 mg/mL ont été ajoutés dans chaque puits. Après 2 heures d'incubation à 37°C, 5% CO2, 200µL de DMSO ont été ajoutés à chaque puits après agitation, et la DO a été lue à 570nm par un spectrophotomètre. Les résultats de ces expériences sont représentés à la Figure 4

Les Figures 4.A et 4.B sont des histogrammes représentant les résultats obtenus lors du test de prolifération réalisé respectivement avec les cellules HT29ctrl et HT29-NRP-2, et avec les cellules Colo320^{siARN-ctrl} et Colo320^{siARN-NRP-2}. L'intensité de coloration (la densité optique « DO ») correspondant à la production de formazan par les cellules est indiquée en ordonnée, et les temps auxquels ont été réalisés les tests de viabilité sont indiqués en abscisse. Pour le panneau A, les barres gris claires représentent les mesures réalisées sur les cellules HT29-NRP-2, les barres gris foncées représentent les mesures réalisées sur les cellules témoins HT29ctrl. Pour le panneau B, les barres gris claires représentent les mesures réalisées sur les Colo320^{siARN-NRP-2}, cellules dont l'expression de NRP-2 est réprimée, les barres gris foncées représentent les mesures réalisées sur les cellules témoins Colo320^{siARN-ctrl} exprimant NRP-2.

La Figure 4.A montre que la DO augmente lorsque les cellules expriment NRP-2, ce qui signifie que l'expression de NRP-2 par les cellules HT29-NRP-2 induit une prolifération et une survie cellulaire supérieure à celle des cellules n'exprimant pas NRP-2.

La Figure 4.B montre que la DO diminue lorsque l'expression de NRP-2 dans les cellules Colo320 est supprimée (cf. barres représentant Colo320^{siARN-NRP-2} en comparaison de Colo320^{siARN-ctrl}). Ce résultat confirme le résultat obtenu avec les cellules HT29 : NRP-2 induit une prolifération et une survie cellulaire supérieure chez les cellules.

L'influence de la neuropiline 2 sur le cycle cellulaire a été étudiée. 50000 cellules HT29 ou HT29-NRP-2 ont été ensemencées dans 1mL de DMEM-10%SVF en plaque 24 puits. 24heures après ensemencement, les cellules ont été trypsinées, lavées 2fois avec 3mL de PBS et reprises dans 1mL d'éthanol 70%. Elles ont été laissées une nuit à 4°C dans l'éthanol 70%. Le lendemain, les cellules sont lavées 2 fois avec 3mL de PBS, digérées avec de la Dnase et marquées avec de l'IP. 30min après, les cellules sont analysées grâce à un cytomètre EPIC'C Altra (Beckman Coulter) et au logiciel d'analyse de cycle Wincycles. Les résultats de cette analyse sont présentés à la Figure 4bis

La Figure 4 bis montre que lorsque les cellules expriment NRP-2, le nombre de cellules en phase G2M et S augmente, alors que le nombre de cellules en phase G1 diminue. Ainsi, l'expression de la neuropiline 2 est associée à une augmentation de la fraction de cellules en phase S et G2M.

L'influence oncogénique de la neuropiline-2 a également été évaluée en comparant chez des souris le développement de xénogreffes de diverses lignées tumorales en fonction de l'expression de la neuropiline-2 dans ces lignées.

Les Figures 5.A, 5.B, 5.C et 5.D sont des photographies de souris ayant été inoculées par voie sous-cutanée respectivement par les cellules HT29ctrl, HT29-NRP-2, Colo320^{siARN-ctrl} et Colo320^{siARN-NRP-2}.

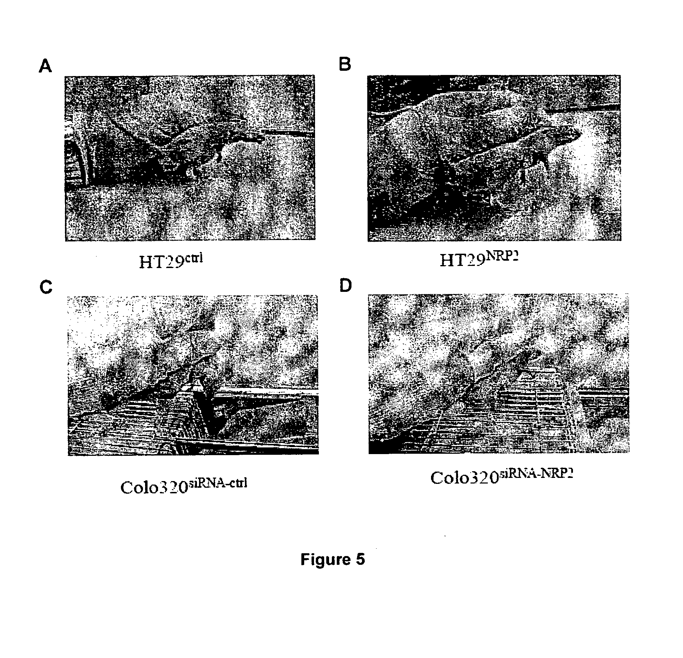

Alors que chez les souris inoculées avec des cellules n'exprimant pas NRP-2 (Fig. 5.A et Fig. 5.D), il n'y a pas de progression anormale de la xénogreffe, une augmentation anormale de la prolifération de la xénogreffe est observée chez les souris inoculées avec des cellules exprimant NRP-2 (Fig. 5.B et Fig. 5.C).

Ces expériences montrent que l'expression de la neuropiline-2 après transfection, ou la répression de cette protéine par ARN interférence, influencent l'oncogenèse des lignées

### Modulation de l'expression de p53 par un siARN ciblant le gène de la NRP-2 humaine.

L'expression de p53, des E-Cadhérines et de la cytokératine 20 dans les xénogreffes de cellules tumorales exprimant ou non la neuropiline-2 (lignée HT29ctrl ou HT29-NRP-2) a été étudiée par immunohistochimie à l'aide d'anticorps spécifiques de chacune des ces trois protéines.

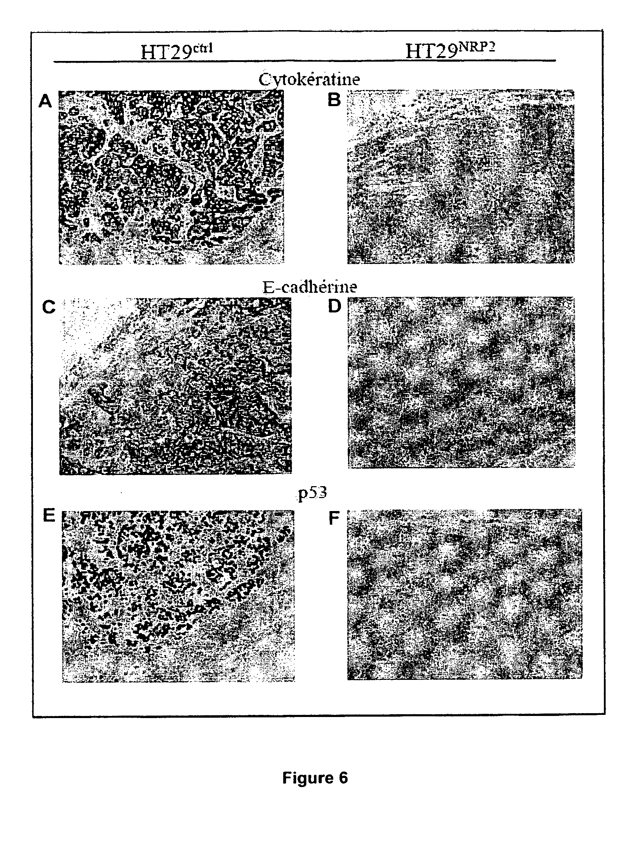

Les Figures 6.A, 6.C et 6.E sont des photographies de coupes de xénogreffes prélevées chez des souris inoculées par la lignée contrôle HT29ctrl, les Figures 6.B, 6.D et 6.F sont des photographies de coupes de xénogreffes prélevées chez des souris inoculées par la lignée exprimant NRP-2 (HT29-NRP-2).

Les coupes de xénogreffes des Figures 6.A et 6.B ont été marquées par un anticorps anti-cytokératine, les coupes de xénogreffes des Figures 6.C et 6.D ont été marquées par un anticorps anti-E-Cadhérine, et les coupes de xénogreffes des Figures 6.E et 6.F ont été marquées par un anticorps anti-p53.

Les Figures 6.A, 6.C et 6.E montrent que les xénogreffes issues de la lignée contrôle HT29ctrl sont fortement marquées par les anticorps anti-cytokératine, anti- E-Cadhérine et anti-p53, alors que les xénogreffes issues de la lignée contrôle HT29-NRP-2 ne sont marquées par aucun des ces anticorps.

Cette étude a montré que la transfection de la neuropiline-2, qui favorise la progression des xénogreffes (Figures 5.A à 5.D), est associée à une perte de l'expression de l'anti-oncogène p53 au niveau des noyaux des cellules tumorales (Figure 6). En outre, l'étude immunohistochimique objective une perte de l'expression des E-Cadhérines et de la cytokératine 20 dans les xénogreffes exprimant la neuropiline-2 (Figure 6), suggérant que l'acquisition de la neuropiline-2 favoriserait la transition épithélio-mésenchymateuse.

L'influence de la neuropiline-2 sur l'expression de p53 a également été étudiée. Pour cela, les lignées HT29-NP2 ou Colo320 ont été traitées soit avec des siARN inhibant la traduction de la neuropiline-2, soit avec des anticorps monoclonaux provenant de l'hybridome ITAC-B1.

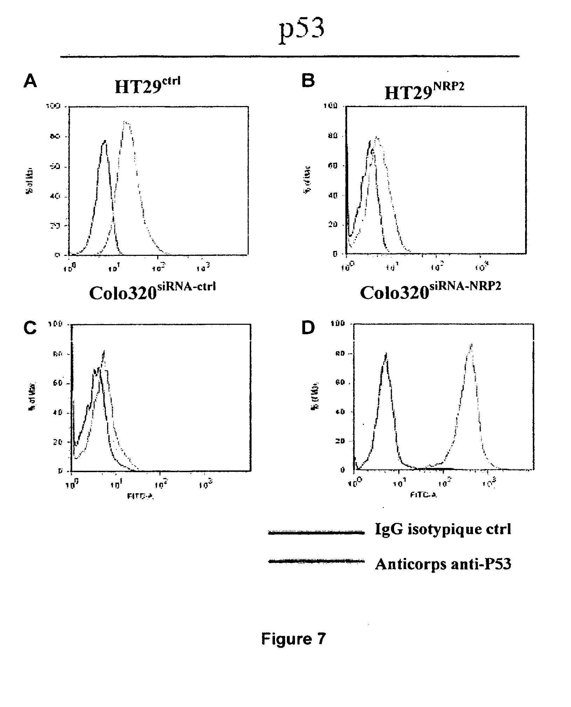

La Figure 7 représente les résultats obtenus par cytométrie en flux pour les cellules HT29-ctrl (panneau A), HT29-NRP-2 (panneau B), Colo320^{siARN-ctrl} (panneau C) et Colo320^{SiARN-NRP-2} (panneau D) marquées à l'aide d'un anticorps anti-p53. Les courbes en traits noirs représentent les résultats du marquage avec un anticorps contrôle, les courbes en traits gris représentent les résultats du marquage avec l'anticorps anti-p53. Le nombre d'évènements (le nombre de cellules) est indiqué en ordonnée, l'intensité de fluorescence (correspondant au marquage des cellules par l'anticorps anti-p53) est représentée en abscisse.

Les panneaux B et C présentant les résultats obtenus pour les lignées exprimant NRP-2, à savoir les panneaux HT29-NRP-2 et Colo320^{siARN-ctrl}, montrent que la protéine p53 n'est pas, ou très peu exprimée (les courbes des populations marquées avec l'anticorps contrôle ou l'anticorps anti-p53 se superposent). En revanche, concernant les panneaux A et D qui présentent les résultats obtenus pour les lignées n'exprimant pas la protéine NRP-2, à savoir les panneaux HT29ctr et Colo320^{siARN-NRP-2}, lors du marquage par l'anticorps anti-p53 (trait gris), il y a un déplacement du pic de distribution des populations cellulaires vers la droite, en comparaison du marquage avec anticorps IgG contrôle (trait noir).

Ces expériences montrent qu'il existe une corrélation négative entre la présence de la neuropiline-2 et celle de p53. En particulier, lorsque colo320, lignée tumorale exprimant constitutivement la neuropiline-2, est traitée par ARN interférence pour inhiber la traduction de cette protéine (lignées Colo320^{siARN-NRP-2}), une restauration de l'expression de p53 dans les lignées tumorales est clairement observée.

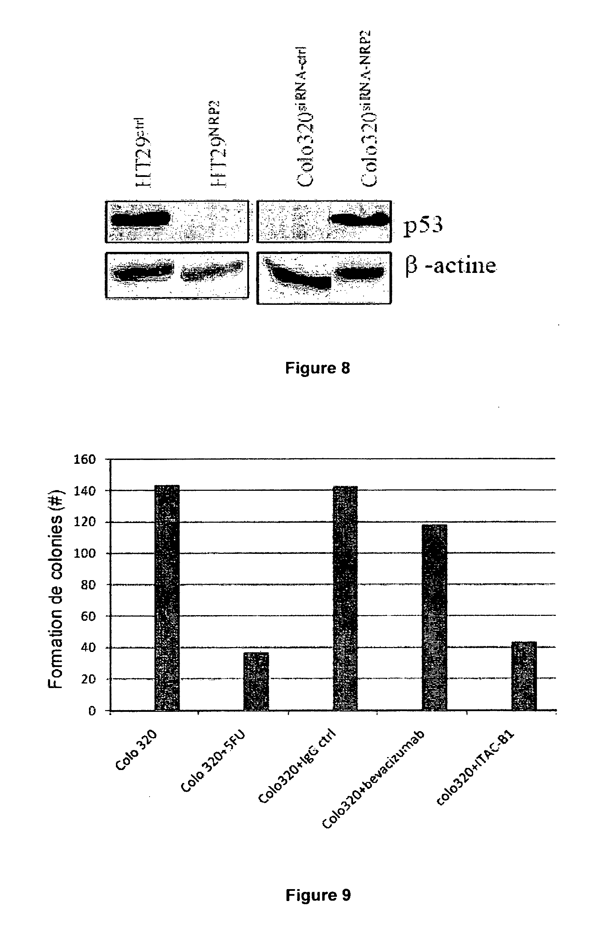

Les extraits protéiques des lignées cellulaires HT29-ctrl, HT29-NRP-2, Col0320^{siARN-ctrl} et Colo320^{siARN-NRP-2} ont également été analysés par transfert de Western à l'aide d'anticorps anti-p53. Après lyse des lignées cellulaires, migration des culots protéiques obtenus sur gel de polyacrylamide 10% (10 µg de protéine par puits, normalisée par rapport à l'immunoblotting de l'actine), puis transfert sur membrane PVDF, la membrane PVDF a été incubée durant une nuit avec l'anticorps primaire p53 (BD Biosciences, mouse anti-human p53) dilué au 1/500. Les membranes, après lavage dans du TBS 0,1% Tween20, ont été incubées pendant 1 heure avec un anticorps secondaire anti-mouse HRP dilué au 1/12500. Les résultats sont représentés sur la Figure 8.

La Figure 8 montre que la protéine p53 est fortement exprimée dans la lignée HT29-ctrl n'exprimant pas NRP-2 alors qu'elle est indétectable dans la lignée HT29-NP2. De la même manière, p53 ne peut être mis en évidence dans la lignée Colo320^{siARN-ctrl} qui exprime NRP-2, alors qu'elle est fortement exprimée dans la lignée Colo320^{siARN-NRP-2} dans laquelle l'expression de NRP-2 est réprimée.

Cette expérience confirme qu'il existe une corrélation négative entre la présence de la neuropiline-2 et l'expression de p53.

Ces données indiquent que l'inhibition de l'expression de la neuropiline-2 permet l'augmentation de l'expression de p53.

### EXEMPLE 4: EFFET DE L'ANTICORPS ITAC-B1 SUR LA CROISSANCE DE CELLULES TUMORALES

### Test de formation de colonies tumorales en milieu gélosé semi-solide

Afin de déterminer si l'anticorps ITAC-B1 possède une activité de neutralisation de la formation de colonies tumorales *in vitro,* des tests de formation de colonies tumorales *in vitro* en milieu gélosé semi-solide contenant de l'agar ont été effectués. Le principe de ce test repose sur la mise en contact de cellules tumorales humaines Colo320 (qui expriment la NRP-2 à leur surface membranaire, et d'autre part sécrètent naturellement du VEGF dans le milieu de culture) et de l'anticorps ITAC-B1. 4000 cellules Colo320 sont ensemencées dans chaque puits d'une plaque 24 puits, en milieu semi-solide contenant de l'agar, et en présence de 10 µg/mL d'anticorps anti-NRP-2 ITAC-B1. A titre de comparaison, le même test est effectué en présence de cytotoxiques d'action prouvée, à savoir du 5-fluoro-uracile (5-FU) utilisé à raison de 50µg/mL ou de l'Avastin® (bevacizumab : anticorps monoclonal humanisé dirigé contre le VEGF) utilisé à raison de 50 µg/mL, ou en présence d'un anticorps contrôle isotypique (anticorps monoclonal IgG1 de souris (BZ1)), utilisé à raison de 10 µg/mL

A 10 jours post ensemencement, le comptage des colonies a été effectué au microscope optique.

La Figure 9 représente les résultats obtenus. Le nombre de colonies formées est indiqué en ordonnée, et les différentes molécules testées sont indiquées en abscisse.

Ces résultats montrent qu'en présence de l'anticorps ITAC-B1 comme en présence de 5 FU, le nombre de colonies est d'environ 40, contre plus de 140 pour le témoin cellules seules ou le contrôle négatif, et environ 120 colonies lorsque les cellules sont cultivées en présence d'Avastin® (bevacizumab).

Il ressort de cette étude *in vitro* que les anticorps anti-NRP-2 ITAC-B1 inhibent presque totalement la formation de colonies tumorales de cellules exprimant la NRP-2 à leur surface, et ceci d'une façon plus efficace que l'Avastin® (bevacizumab).

La neuropiline-2 étant un co-récepteur du VEGF, il a été vérifié si l'activité d'ITAC-B1 était dépendante d'une interaction entre NRP-2 et le VEGF produit par les cellules Colo320. Dans ce but, les tests de formation de colonies de cellules Colo 320 ont été effectués dans les conditions décrites ci-dessus, et en présence d'anticorps ITAC-B1 (10 µg/mL), de bevacizumab (50µg/mL) ou d'anticorps contrôle (10 µg/mL), utilisés isolément ou en association.

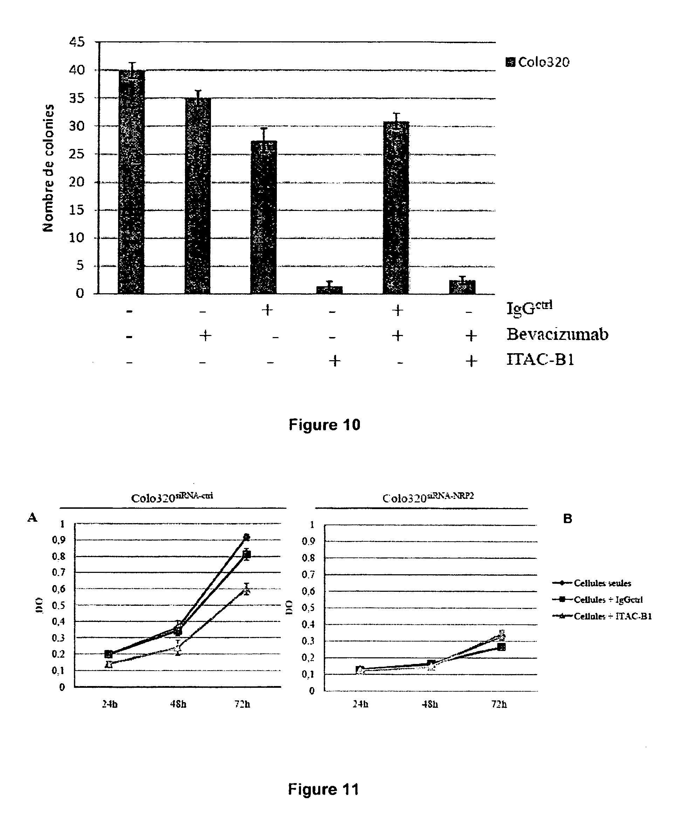

Les résultats sont présentés sur la Figure 10: l'ordonnée représente le nombre de colonies formées ; en abscisse les anticorps présents dans le milieu de culture sont indiqués parun «+».

La Figure 10 montre que la présence de bevacizumab dans le milieu de culture affecte très peu la prolifération des cellules Colo 320 : le nombre de colonies observées en présence de l'anticorps contrôle, du bevacizumab, ou de l'association de ces 2 anticorps est similaire, et ne diffère que peu de celui observé avec le témoin cellules seules. En revanche, le nombre de colonies observé en présence de l'anticorps ITAC-B1 est fortement diminué par rapport au témoin cellules seules ; en outre lorsque le milieu de culture contient l'anticorps ITAC-B1 et le bevacizumab (qui neutralise VEGF-A), le nombre de colonies est identique à celui observé lorsque le milieu contient l'anticorps ITAC-B1 seul. Ce résultat indique qu'ITAC-B1 inhibe aussi efficacement la prolifération cellulaire en présence de VEGF qu'en l'absence de VEGF, et donc que l'effet thérapeutique de ITAC-B1 est indépendant de l'interaction NRP-2/VEGF.

### Test MTT de prolifération de cellules tumorales :

L'effet de l'anticorps ITAC-B1 sur la prolifération de cellules tumorales humaines présentant la NRP-2 à leur surface membranaire, a également été étudié en utilisant le test MTT, comme décrit à l'Exemple 3 ci-dessus.

4000 cellules par puits de cellules Colo320^{siARN-ctrl} (cellules contrôle transfectées par un vecteur vide) ou de cellules Colo320^{siARN-NRP-2} (transfectées par un siARN ciblant la NRP-2) , dans 100µL de milieu DMEM contenant 10% de SVF inactivé, ont été ensemencées en plaque Maxisorp 96 puits. Après adhérence des cellules, 5µg/mL d'anticorps anti-NRP-2 ITAC-B ou 5µg/mL µg/mL d'anticorps contrôle ont été ajoutés. Après 24, 48, ou 72 heures de culture, 10 µL de MTT reconstitué à 5 mg/mL dans du PBS ont été ajoutés à chaque puits de culture. Les plaques ont ensuite été incubées 3 heures dans le noir, à 37°C, et 5% de CO₂, centrifugées, puis le surnageant a été éliminé. 200µL de DMSO ont alors été ajoutés dans chaque puits. La densité optique a été lue dans l'heure, à 570 nm, après agitation de la plaque. Le test a été effectué en triplicate. Les résultats sont présentés sur la Figure 11.

Les panneaux A et B de la Figure 11 représentent respectivement les résultats obtenus lors du test de prolifération réalisé avec les cellules Colo320^{siARN-ctrl} ou Colo320^{siARNNRP-2}. La densité optique « DO ») traduisant la production de formazan par les cellules est indiquée en ordonnée, et la durée de culture est indiquée en abscisse. Le témoin cellules seules est représenté par 0, les cellules cultivées en présence de l'anticorps contrôle sont représentées par ■, et les cellules cultivées en présence de l'anticorps ITAC-B1 sont représentées par Δ.

Ces résultats montrent que dans le cas des cellules Colo320^{siARN-ctrl} qui expriment NRP-2, la prolifération en présence de l'anticorps ITAC-B1 est inférieure à celle des cellules seules et celle observée en présence de l'anticorps contrôle. En revanche, dans le cas des cellules Colo320^{siARN-NRP-2} la prolifération en présence de l'anticorps ITAC-B1 est identique à celle des cellules témoin et à celle observée en présence de l'anticorps contrôle. Les anticorps ITAC-B1 ralentissent donc spécifiquement la prolifération des cellules présentant NRP-2 à la surface (cellules Colo320^{siARN-ctrl}) et non celle des cellules n'exprimant pas NRP-2 (cellules Colo320^{SiARN-NRP-2}).

### EXEMPLE 5: L'ANTICORPS ITAC-B1 POSSEDE LA CAPACITE D'INDUIRE L'APOPTOSE DE CELLULES TUMORALES EXPRIMANT LA NEUROPILINE-2

Afin de déterminer la capacité de l'anticorps ITAC-B1 à induire l'apoptose de cellules tumorales exprimant la NRP-2, un test *in vitro* d'apoptose Annexine V-APC a été réalisé (BD Pharmingen, San Diego, CA). Ce test repose sur l'externalisation de la phosphatidylsérine par les cellules apoptotiques, et sur la fixation de l'Annexine V-APC sur cette molécule.

### I - Effet pro-apoptotique d'ITAC-B1 seul

1mL de milieu DMEM contenant 10% de SVF inactivé et 100000 cellules HT29-NRP-2 ou 100000 cellules HT29ctrl ont été ensemencées par puits dans une plaque Nunc 24 puits. Après adhérence des cellules (3heures), trois concentrations d'anticorps anti-NRP-2 ITAC-B (0,5µg/mL, 1µg/mL et 5µg/mL) ont été testées dans différents puits. En parallèle, des témoins cellules seules ont été réalisés, ainsi que des contrôles négatifs (anticorps de souris IgG1 anti-human (BZ1) aux mêmes concentrations que l'anticorps ITAC-B1).

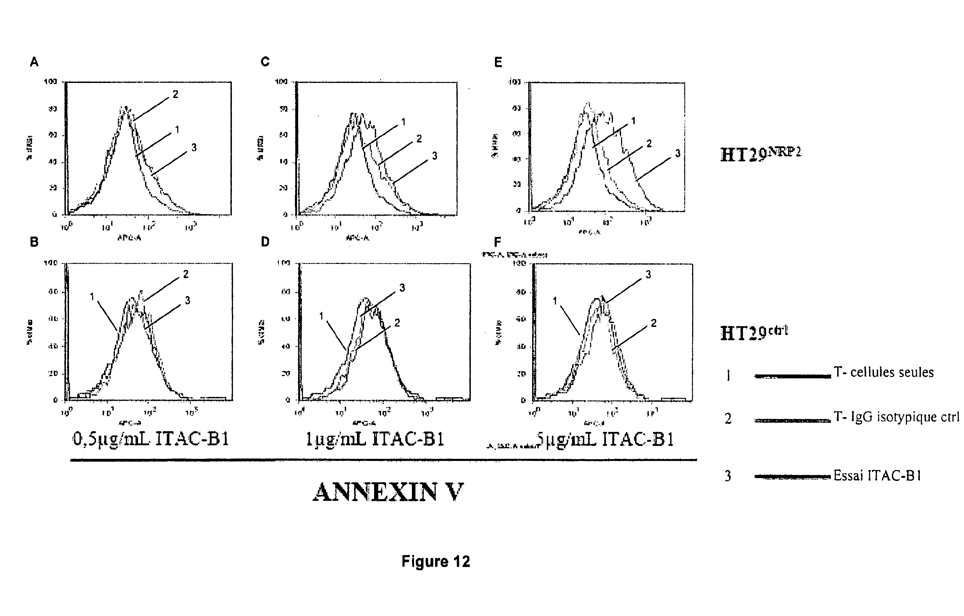

Après 16 heures d'incubation, le surnageant de culture a été aspiré et 500µL de trypsine-EDTA ont été ajoutés dans chaque puits, et laissés en contact pendant 10 minutes. Lorsque les cellules ont commencé à se détacher, 500µL de milieu DMEM-10% SVF ont été ajoutés par puits. Les cellules ont alors été centrifugées, lavées 2 fois dans du PBS puis reprises dans 300µL de Tampon Binding Buffer 1X (fourni dans le kit), puis 5µL d'Annexine V-APC ont ensuite été ajoutés à 100µL de cette solution. L'analyse par cytométrie en flux des populations de cellules marquées par l'Annexine V-APC en fonction des concentrations en anticorps ITAC-B1, et en anticorps contrôle, est présenté à la Figure 12.

Pour chacun des panneaux de la Figure 12, le nombre d'évènements (nombre de cellules) est indiqué en ordonnée et l'intensité de fluorescence (correspondant au marquage des cellules par l'annexine V-APC) est représentée en abscisse. Les panneaux A, C et E représentent les expériences réalisées avec les cellules HT29-NRP-2, les panneaux B, D et F représentent les expériences réalisées avec les cellules HT29ctrl. Les expériences pour lesquelles la concentration en anticorps ITAC-B1 est de 0,5µg/mL sont représentées aux panneaux A et B de la Figure 12. Les expériences pour lesquelles la concentration en anticorps ITAC-B1 est de 1µg/mL sont représentées aux panneaux C et D de la Figure 12, et les expériences pour lesquelles la concentration en anticorps ITAC-B1 est de 5µg/mL sont représentées aux panneaux E et F de la Figure 12. Pour chaque panneau, la courbe de distribution (1) représente les cellules seules, la courbe de distribution (2) représente les cellules cultivées en présence d'un anticorps isotypique murin contrôle et la courbe de distribution (3) représente les cellules cultivées en présence de l'anticorps ITAC-B1.

On constate, pour les panneaux C et E représentant les expériences réalisées avec les cellules HT29-NRP-2 exprimant NRP-2 un déplacement vers la droite du pic de distribution correspondant respectivement aux concentrations 1 et 5 µg/mL d'anticorps ITAC-B1. Ce déplacement du pic de distribution n'est pas observé pour les témoins cellules seules et anticorps contrôle. Ce résultat indique que l'apoptose est spécifiquement induite chez les cellules exprimant NRP-2 à partir de 1 µg/mL d'anticorps ITAC-B1 dans le milieu de culture.

En ce qui concerne les expériences correspondantes réalisées avec les cellules HT29ctrl qui n'expriment pas NRP-2, les courbes représentant les conditions cellules seules, témoins anticorps contrôle et anticorps ITAC-B1 se superposent, indiquant que l'anticorps ITAC-B1 n'entraine pas l'apoptose de cellules n'exprimant pas NRP-2.

On constate que l'anticorps ITAC-B1 induit l'apoptose de cellules exprimant la NRP-2 à leur surface cellulaire, à une dose égale ou supérieure à 1µg/mL. L'apoptose induite par ITAC-B1 est dose-dépendante puisqu'une concentration plus importante d'ITAC-B1 entraîne une apoptose plus importante.

Ces expériences montrent donc que les anticorps ITAC-B1 induit spécifiquement l'apoptose de cellules tumorales présentant la NRP-2 (HT29-NRP-2) à leur surface membranaire, sans induire l'apoptose de cellules ne présentant pas la NRP-2 (HT29).

### II - Effet pro-apoptotique d'ITAC-B1 en combinaison avec d'autres agents anti-cancéreux

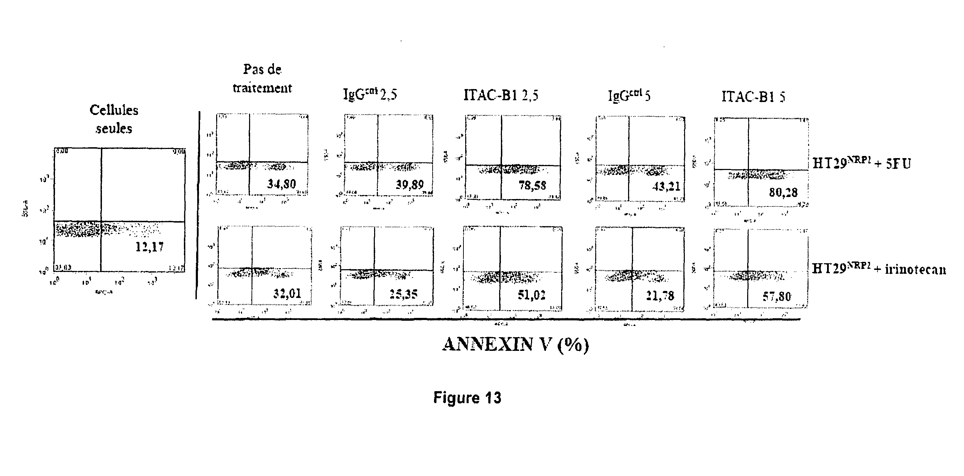

Des expériences similaires d'évaluation de la capacité d'ITAC-B1 à induire l'apoptose de cellules exprimant NRP-2 ont été réalisées avec des cellules cultivées en présence soit de 5FU (5 fluoro-uracile), soit d'irinotecan, deux anticancéreux couramment utilisés en chimiothérapie. Dans cette série d'expériences, les concentrations en anticorps ITAC-B1 de 2,5µg/mL et 5µg/mL ont été testées. En parallèles, des témoins cellules seules ont été réalisés, ainsi que des contrôles avec l'anticorps BZ1 aux concentrations 2,5µg/mL et 5µg/mL. Le 5FU et l'irinotecan sont utilisés à 10µg/mL. Les résultats de ces expériences sont représentés à la Figure 13.

Les panneaux de la Figure 13 sont des représentations en nuage de points de l'analyse par cytométrie en flux. Pour chacun des panneaux, l'intensité de fluorescence (correspondant au marquage des cellules par l'annexine V-APC) est représentée en abscisse. L'ordonnée indique la granulosité des cellules (Side Scatter ou SSC). Les conditions de traitement par les anticorps sont indiquées au dessus des panneaux, et le traitement par le 5FU ou l'irinotecan est indiqué à droite des panneaux. Le pourcentage de cellules marquées par l'annexine V-APC, (correspondant au pourcentage de cellules entrées en apoptose), est indiqué en bas à droite de chaque panneau.

Les cellules seules présentent environ 12% d'apoptose, et les cellules cultivées en présence de 5FU ou d'irinotecan seuls, ou associés à l'anticorps contrôle BZ1 présentent entre 34 et 43% environ d'apoptose dans le cas du 5FU, et entre 21 et 35% d'apoptose dans celui de l'irinotecan.

L'apoptose chez les cellules cultivées conjointement en présence de 5FU et de l'anticorps ITAC-B1 est de l'ordre de 78,5% pour une concentration d'ITAC-B1 dans le milieu de 2,5µg/mL, et de l'ordre de 80% pour une concentration d'ITAC-B1 de 5µg/mL.

Pour les cellules cultivées en présence d'irinotecan et d'ITAC-B1, l'apoptose est de l'ordre de 51% pour une concentration d'anticorps ITAC-B1 de 2,5µg/mL, et de l'ordre de 58% pour une concentration de 5µg/mL.

Ces résultats mettent donc en évidence une synergie d'action entre les anticorps ITAC-B1 et les molécules anticancéreuses, la combinaison 5FU/ITAC-B1 étant la plus efficace dans les conditions testées.

### III - L'effet pro-apoptotique d'ITAC-B1 est corrélé à l'expression de p53

Afin de corréler l'induction d'apoptose à l'expression de p53, les cellules Colo320siARN-ctrl ont été prétraitées 18h avec un inhibiteur chimique de p53, la pifithrin-α, à une dose de 27µM (PFTα, Sigma). Après prétraitement, les cellules sont lavées 2 fois avec 3mL de PBS et mises en plaque 24 puits, à raison de 100000 cellules/puits dans 1mL de RPMI-10% SVF. Des cellules non prétraitées sont mises dans les mêmes conditions. Les cellules sont incubées pendant 5h avec un anticorps isotypique murin contrôle (BZ1) ou ITAC-B1 à 20µg/mL et l'apoptose est mesurée comme décrit ci-dessus.

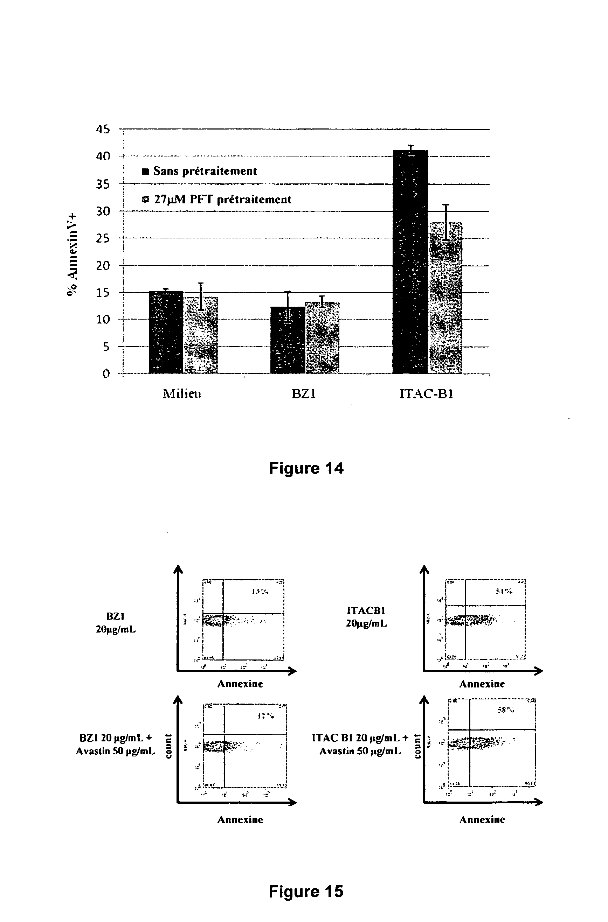

La Figure 14 représente les résultats des expériences d'induction de l'apoptose réalisées avec les cellules Colo320siARN-ctrl prétraitées ou non par la PFTα. Le pourcentage de cellules marquées par l'annexine V-APC est indiqué en ordonnée, les conditions de cultures des cellules sont indiquées en abscisse (témoin milieu, témoin milieu additionné d'un anticorps contrôle isotypique -BZ1-, essai milieu additionné de l'anticorps ITAC-B1). Les résultats des essais réalisés avec des cellules prétraitées par la PFTα sont représentés en gris clair, ceux des essais réalisés avec des cellules non prétraitées sont représentés en gris foncé.

Alors que dans les témoins milieu et milieu additionné d'anticorps contrôle le marquage à l'annexine V-APC est similaire que les cellules soient traitées ou non par la PFTα, on constate que les cellules cultivées en présence d'anticorps ITAC-B1 sont plus faiblement marquées lorsqu'elles sont prétraitées avec la PFTα (environ 28% de marquages contre 41% pour les cellules non prétraitées).

Ces résultats montrent que le prétraitement par la PFTα prévient l'apoptose dépendante d'ITAC-B1. L'apoptose induite par ITAC-B1 semble donc dépendre de l'expression de p53.

### IV - L'effet pro-apoptotique d'ITAC-B1 est indépendant du VEGF

Afin de déterminer si l'effet apoptotique observé lors du traitement des cellules par l'anticorps ITAC-B1 était dépendant de la liaison du VEGF à NRP-2, les cellules tumorales HT29-NP2 exprimant NRP-2 (et qui comme les cellules Colo320 sécrètent naturellement du VEGF dans le milieu de culture) ont été cultivées en présence de l'anticorps monoclonal humanisé anti-VEGF Avastin® (bevacizumab) à 50µg/mL, puis les cellules ont été incubées en présence d'un anticorps isotypique murin contrôle (BZ1) ou d'ITAC-B1 à 20µg/mL durant 6 heures. L'apoptose induite a alors été mesurée par marquage à l'annexine V-APC comme décrit ci-dessus. Les résultats sont présentés sur la Figure 15.

Les panneaux de la Figure 15 sont des représentations en nuage de points de l'analyse par cytométrie en flux. Pour chacun des panneaux, l'intensité de fluorescence (correspondant au marquage des cellules par l'annexine V-APC) est représentée en abscisse. L'ordonnée indique la granulosité des cellules. Les conditions de traitement par les anticorps et l'Avastin® sont indiquées à gauche des panneaux. Le pourcentage de cellules marquées par l'annexine V-APC, correspondant au pourcentage de cellules entrées en apoptose, est indiqué en haut à droite de chaque panneau.

Les cellules cultivées en présence de l'anticorps contrôle BZ1, avec ou sans Avastin®, présentent respectivement 12 et 13% d'apoptose. L'apoptose chez les cellules HT29-NP2 cultivées en présence de l'anticorps ITAC-B1 est de l'ordre de 51%, et d'environ 58% lorsque les cellules ont été préalablement traitées par l'Avastin®.

Ces résultats montrent clairement que la neutralisation du VEGF n'altère pas la capacité de l'anticorps ITAC-B1 à induire l'apoptose, ce qui confirme que les propriétés pro-apoptotiques de l'anticorps ITAC-B1 sont indépendantes du VEGF.

### EXEMPLE 6: ITAC-B1 N'INFLUENCE PAS LA PHOSPHORYLATION DU RECEPTEUR VEGFR1 OU DE LA PROTEINE AKT.

L'effet éventuel de l'anticorps ITAC-B1 sur le degré de phosphorylation d'un récepteur au VEGF (VEGFR1), ainsi que sur la phosphorylation de la protéine AKT qui est activée par l'intermédiaire des récepteurs du VEGF a été étudié sur des cellules tumorales HT29-NRP-2 et Colo320^{siARN-ctrl}.

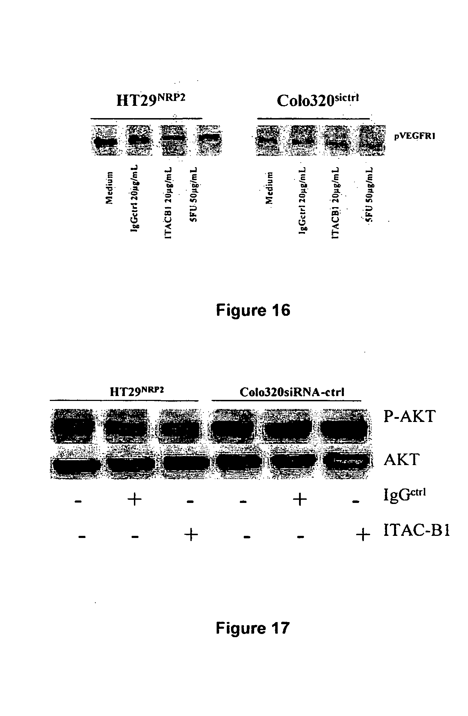

Les cellules ont été cultivées pendant 24 heures en milieu RPMI + 10% SVF en présence de l'anticorps contrôle BZ1 à 20µg/mL, d'ITAC-B1 à 20µg/mL ou de 5FU à 50µg/mL. Un témoin cellules non-traitées a été ajouté (témoin « medium »). Les cellules ont ensuite été isolées et lysées, et la phosphorylation de VEGFR-1 et d'AKT a été évaluée par Western Blot sur les extraits protéiques cellulaires, en utilisant des anticorps dirigés contre le VEGFR1 non-phosphorylé (anticorps polyclonal de lapin anti-VEGFR1) ou phosphorylé (anticorps polyclonal de lapin anti-phospho-VEGFR1^{Tyrl213}, R&D system) ou des anticorps dirigés contre AKT non-phosphorylé (anticorps polyclonal de lapin C67E7, Cell Signaling technology) ou phosphorylé (anticorps polyclonal de lapin anti-phospho-AKT^{Ser473} DE-9, Cell Signaling technology). Les résultats sont représentés sur les Figures 16 et 17.

Ces résultats montrent que le statut de phosphorylation de VEGFR, ainsi que la quantité d'AKT et son statut de phosphorylation sont similaires, que les cellules soient traitées ou non par ITAC-B1, démontrant que les effets de cet anticorps ne sont pas liés à la voie de signalisation VEGF/VEGFR.

### EXEMPLE 7: RECHERCHE D'UNE EVENTUELLE INTERACTION ENTRE LE VEGF ET L'EPITOPE DE LA NEUROPILINE 2 RECONNU PAR ITAC-B1.

Afin de mettre en évidence une éventuelle compétition entre le VEGFa et ITAC B1 pour la reconnaissance de la neuropiline 2, des cellules tumorales HT29-NRP-2 exprimant la neuropiline 2 ont été préincubées avec ou sans VEGF (1000 ng/mL) pendant 15 minutes. Ces cellules ont ensuite été incubées avec l'anticorps ITAC B1, ou avec un anticorps contrôle isotypique (anticorps BZ1), et analysés en cytométrie de flus comme décrit dans l'Exemple 2 ci-dessus.

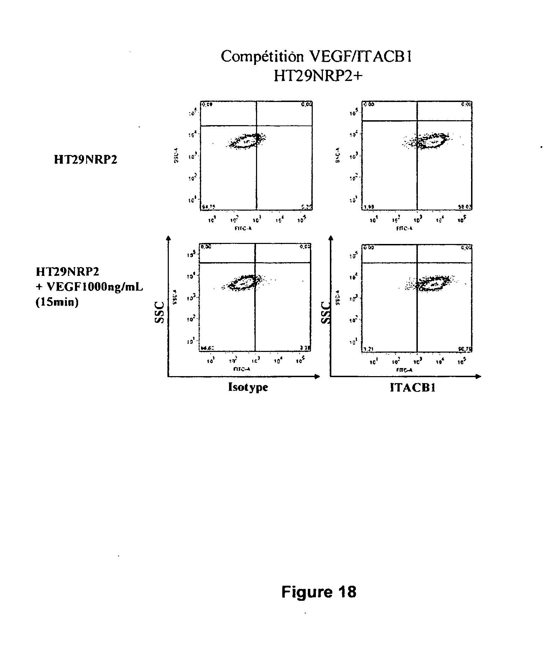

Les résultats sont illustrés par la Figure 18. Ces résultats montrent que la présence du VEGFA ne prévient pas la fixation de ITAC B1 sur les cellules tumorales.

### EXEMPLE 8: LES ANTICORPS ITAC-B1 ET ITAC-B2 ONT LA CAPACITE D'INDUIRE L'EXPRESSION DE LA PROTEINE P53

Après avoir montré que la neuropiline-2 a la capacité d'altérer l'expression de p53 dans la lignée HT29 et de la restaurer dans la lignée Colo320^{siARN-NRP-2}, l'influence de l'anticorps ITAC-B1 sur l'expression de p53 dans des lignées exprimant la neuropiline-2, comme HT29-NRP-2 et Colo320siARN-ctrl, a été étudiée.

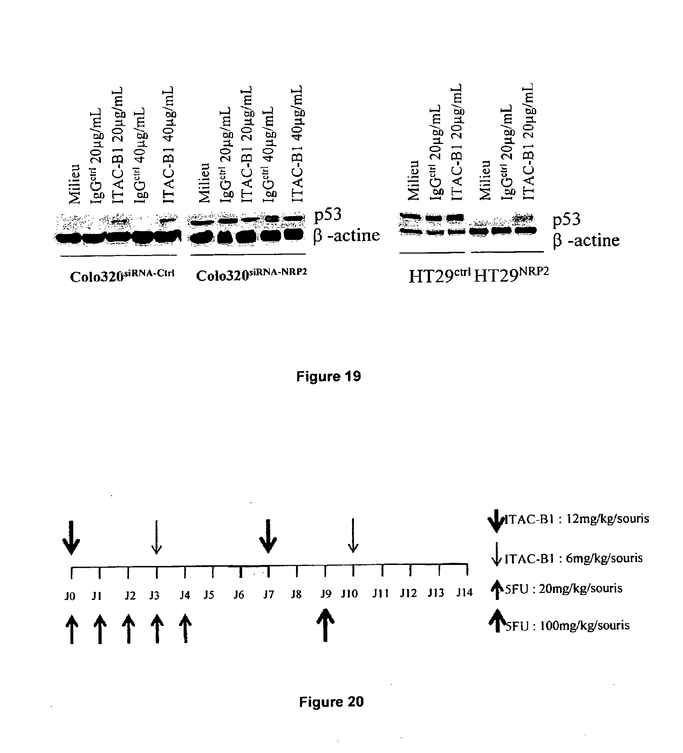

Les cellules tumorales HT29, HT29-NRP-2, Colo320^{siARN-ctrl} et Colo320^{siARNNRP-2} ont été exposées à 20µg/ml d'anticorps ITAC-B1 ou d'anticorps contrôle, et cultivées pendant 48 heures. Après lyse des lignées cellulaires, migration des culots protéiques obtenus sur gel de polyacrylamide 10% (10 µg de protéine par puits), puis transfert sur membrane PVDF, la membrane a été incubée durant une nuit avec l'anticorps primaire anti-p53 (BD Biosciences, mouse anti-human p53) dilué au 1/500. Les membranes, après lavage dans du TBS 0,1% Tween20, ont été incubées pendant 1 heure avec un anticorps secondaire anti IgG-HRP de souris dilué au 1/6000. Les résultats sont représentés à la Figure 19.

Ces résultats montrent que la protéine p53 est fortement exprimée dans les lignées HT29-ctrl et Colo320^{siARN-NRP-2} n'exprimant pas NRP-2, que l'anticorps ITAC-B1 soit présent ou non dans le milieu. Lorsque les lignées HT29-NRP-2 et Colo320^{siARN-ctrl} exprimant NRP-2 sont cultivées seules ou en présence de l'anticorps contrôle, p53 n'est pas détectée, alors que la présence de l'anticorps ITAC-B1 dans le milieu de culture restaure en partie l'expression de p53. Ces résultats montrent donc que le traitement de cellules exprimant NRP-2 à leur surface membranaire (HT29-NP2 et Colo320^{siARN-ctrl}) par ITAC-B1 aboutit à la restauration de l'expression de p53 dans les lignées tumorales.

Cette expérience confirme qu'il existe une corrélation négative entre la présence de la neuropiline-2 et de p53, et que ITAC-B1 possède la capacité originale de pouvoir moduler le niveau d'expression de p53.

### EXEMPLE 7 : UTILISATION DE L'ANTICORPS ITAC-B1 POUR POTENTIALISER L'EFFET DE TRAITEMENTS ANTI-NEOPLASIQUES IN VIVO

Des cellules tumorales HT29-NRP-2 ont été injectées en sous-cutané à 20 souris immunodéficientes, à raison de 1.10⁶ cellules par souris, au niveau du flanc droit. 10 jours après l'injection, les tumeurs mesurent environ 5mm x 5mm. Quatre groupes de 3 souris possédant des tumeurs comparables ont été formés : chaque groupe a reçu par voie intrapéritonéale soit du PBS (Groupe contrôle A), soit l'anticorps ITAC-B1 (Groupe B), soit du 5FU (Groupe C), soit du 5FU et l'anticorps ITAC-B1 (Groupe D).

Le protocole de traitement de chimiothérapie est schématisé sur la figure 20.

Conformément au protocole décrit ci-dessus, le Groupe B a été inoculé avec ITAC-B 1 à J0 (12mg/kg), J3 (6mg/kg), J7 (12mg/kg) et J10 (6mg/kg). Le Groupe C a été inoculé avec 20mg/kg de 5FU de J0 à J4 puis avec 100mg/kg à J9. Le Groupe D a été inoculé avec ITAC-B1 à J0 (12mg/kg), J3 (6mg/kg), J7 (12mg/kg) et J10 (6mg/kg) et avec 20mg/kg de 5FU de J0 à J4 et 100mg/kg de 5FU à J9.

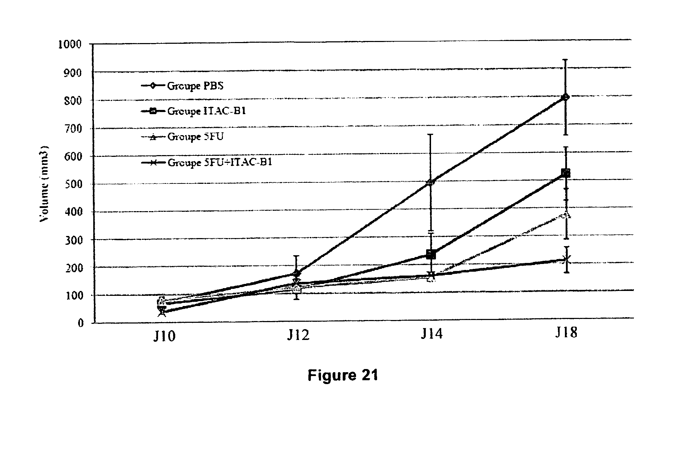

Les tumeurs ont été mesurées 2 fois par semaine, et le calcul du volume des tumeurs a été effectué avec la formule: V (mm³) = d² x D / 2. Les résultats de ces mesures sont présentés dans la Figure 21.

La Figure 21 représente le volume des tumeurs en fonction du temps. Le Groupe A (témoin PBS) est représenté par (0), le Groupe B (souris inoculées avec l'anticorps ITAC-B1) est représenté par (■), le Groupe C (souris inoculées avec le 5FU) est représenté par (Δ), et le Groupe D (souris inoculées à la fois avec l'anticorps ITAC-B1 et avec le 5FU) est représenté par (x).

On observe une réduction du volume des tumeurs à partir de J14 chez les souris inoculées avec l'anticorps ITAC-B1 ou avec du 5FU. Cette réduction est plus marquée lors du traitement combiné 5FU/ITAC-B1.

Ces résultats montrent que l'anticorps ITAC-B1 est capable de ralentir *in vivo* la croissance tumorale. En outre, puisque les souris traitées par du 5FU associé à ITAC-B1 connaissent le ralentissement de croissance tumorale le plus important, par rapport aux souris traitées par 5FU seul ou ITAC-B1 seul, ces résultats montrent clairement que l'utilisation de l'anticorps ITAC-B1 potentialise l'efficacité du 5-fluorouracile.

## Revendications

1. Anticorps anti-neuropiline-2 humaine, **caractérisé en ce qu'**il est choisi parmi :
i) un anticorps comprenant le domaine variable de la chaîne lourde et le domaine variable de la chaîne légère de l'anticorps ITAC-B1, lesdits domaines étant respectivement définis par les séquences SEQ ID NO: 2 et SEQ ID NO: 4 ;
ii) un anticorps comprenant le domaine variable de la chaîne lourde et le domaine variable de la chaîne légère de l'anticorps ITAC-B2, lesdits domaines étant respectivement définis par les séquences SEQ ID NO: 6 et SEQ ID NO: 8.

2. Ligand de la neuropiline-2 humaine dérivé d'un anticorps selon la revendication 1, **caractérisé en ce qu'**il comprend au moins les CDR1, CDR2 et CDR3 de la chaîne lourde et les CDR1, CDR2 et CDR3 de la chaîne légère de l'anticorps ITAC-B1, dans lequel lesdits CDR sont définis respectivement par les séquences SEQ ID NO : 9 à 14, ou de l'anticorps ITAC-B2, dans lequel lesdits CDR sont définis respectivement par les séquences SEQ ID NO : 15 à 20.

3. Ligand de la neuropiline-2 humaine selon la revendication 2, **caractérisé en ce qu'**il s'agit d'un anticorps humanisé.

4. Utilisation d'un anticorps selon la revendication 1, ou d'un ligand selon une quelconque des revendications 2 ou 3, pour l'obtention d'un médicament anti-tumoral.

5. Utilisation selon la revendication 4, **caractérisée en ce que** ledit médicament induit l'apoptose des cellules tumorales exprimant la neuropiline-2 humaine en augmentant l'expression de p53 dans lesdites cellules.

## Patentansprüche

1. Antikörper gegen humanes Neuropilin-2, **dadurch gekennzeichnet, dass** er ausgewählt ist aus:
i) einem Antikörper, der die variable Domäne der schweren Kette und die variable Domäne der leichten Kette des Antikörpers ITAC-B1 umfasst, wobei die Domänen durch die Sequenzen SEQ ID NO: 2 bzw. SEQ ID NO: 4 definiert sind;
ii) einem Antikörper, der die variable Domäne der schweren Kette und die variable Domäne der leichten Kette des Antikörpers ITAC-B2 umfasst, wobei die Domänen durch die Sequenzen SEQ ID NO: 6 bzw. SEQ ID NO: 8 definiert sind.

2. Ligand des humanen Neuropilin-2, der von einem Antikörper gemäß Anspruch 1 abgeleitet ist, **dadurch gekennzeichnet, dass** er wenigstens die CDR1, CDR2 und CDR3 der schweren Kette und die CDR1, CDR2 und CDR3 der leichten Kette des Antikörpers ITAC-B1 umfasst, wobei die CDR jeweils durch die Sequenzen SEQ ID NO: 9 bis 14 definiert sind, oder des Antikörpers ITAC-B2, indem die CDR entsprechend durch die Sequenzen SEQ ID NO: 15 bis 20 definiert sind.

3. Ligand des humanen Neuropilin-2 gemäß Anspruch 2, **dadurch gekennzeichnet, dass** es sich um einen humanisierten Antikörper handelt.

4. Verwendung eines Antikörpers gemäß Anspruch 1 oder eines Liganden gemäß einem der Ansprüche 2 oder 3 zur Herstellung eines Antitumor-Medikamentes.

5. Verwendung gemäß Anspruch 4, **dadurch gekennzeichnet, dass** das Medikament die Apoptose der Tumorzellen, die humanes Neuropilin-2 exprimieren, induziert, indem es die Expression von p53 in den genannten Zellen erhöht.

## Claims

1. Anti-human neuropilin-2 antibody, **characterised in that** it is selected from:
i) an antibody comprising the variable domain of the heavy chain and the variable domain of the light chain of the ITAC-B1 antibody, said domains being defined by sequences SEQ ID NO: 2 and SEQ ID NO: 4 respectively,
ii) an antibody comprising the variable domain of the heavy chain and the variable domain of the light chain of the ITAC-B2 antibody, said domains being defined by sequences SEQ ID NO: 6 and SEQ ID NO: 8 respectively.

2. Ligand for human neuropilin-2 derived from an antibody according to claim 1, **characterised in that** it comprises at least the heavy chain CDR1, CDR2 and CDR3 and the light chain CDR1, CDR2 and CDR3 of the ITAC-B1 antibody in which said CDRs are defined by sequences SEQ ID NOS: 9 to 14 respectively, or those of the ITAC-B2 antibody in which said CDR's are defined by sequences SEQ ID NOS: 15 to 20 respectively.

3. Ligand for human neuropilin-2 according to claim 2, **characterised in that** it is a humanised antibody.

4. Use of an antibody according to claim 1, or of a ligand according to either of claims 2 and 3, for obtaining an anti-tumour medicament.

5. Use according to claim 4, **characterised in that** said medicament induces apoptosis of the tumour cells expressing human neuropilin-2 by increasing p53 expression in said cells.
